(19) Europäisches Patentamt
European Patent Office
Office européen des brevets

(11) EP 2 579 024 A1

(12) EUROPEAN PATENT APPLICATION
published in accordance with Art. 153(4) EPC

(43) Date of publication:
10.04.2013 Bulletin 2013/15

(51) Int Cl.:
G01N 21/64 (2006.01)        G01N 33/53 (2006.01)
G01N 33/536 (2006.01)

(21) Application number: 11789480.8

(22) Date of filing: 03.06.2011

(86) International application number:
PCT/JP2011/003140

(87) International publication number:
WO 2011/152067 (08.12.2011 Gazette 2011/49)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR

(30) Priority: 24.05.2011  JP 2011115473
04.06.2010  JP 2010129445

(71) Applicant: FUJIFILM Corporation
Tokyo 106-8620 (JP)

(72) Inventor: KIMURA, Toshihito
Ashigarakami-gun
Kanagawa 258-8538 (JP)

(74) Representative: HOFFMANN EITLE
Patent- und Rechtsanwälte
Arabellastrasse 4
81925 München (DE)

(54) DEVICE FOR DETECTING BIOMOLECULE AND METHOD FOR DETECTING BIOMOLECULE

(57) [Objective]
To provide a biological molecule detecting apparatus capable of highly sensitive measurements.
[Constitution]
A laser beam is emitted onto fluorescent molecules within a solution to orient the fluorescent molecules. The direction in which the laser beam is emitted is switched to switch the direction of the transition moment of the fluorescent particles to be parallel to or perpendicular to the vibrating direction of excitation light, which is linearly polarized. Thereby, the fluorescent molecules of free molecules and binding molecules can be switched between an excited state and a non excited state. There is a difference in the speeds at which the orientation directions change for molecules which have undergone antigen antibody reactions and for molecules which have not undergone antigen antibody reactions. Therefore, the fluorescence contributed by fluorescent molecules associated with free molecules and the fluorescence contributed by fluorescent molecules associated with binding molecules can be calculated respectively, to measure the concentration of a detection target substance with high sensitivity.

FIG.5

EP 2 579 024 A1

**Description**

Technical Field

The present invention is related to technology for detecting detection target substances within solutions. Particularly, the present invention is related to a biological molecule detecting apparatus and a biological molecule detecting method capable of detecting biological molecules, viruses, nucleic acids, proteins, and germs within samples.

Background Art

Recently, biological molecule detecting methods, in which physicians or technicians detect biological molecules at points of care, immediately obtain measurement results, and utilize the measurement results for diagnosis and treatment, are being focused on. Biological molecule detecting methods are methods for selectively detecting only detection target substances from within bodily fluids such as blood, urine, and sweat, by the high selectivity of specific reactions such as antigen antibody reactions. Such biological molecule detecting methods are particularly widely employed to detect, inspect, quantify, and analyze small amounts of biological molecules, such as viruses, nucleic acids, proteins, and germs.

Radioimmunoassay is a biological molecule detecting method which is in practical use. Radioimmunoassay employs antigens or antibodies labeled with isotopes, and detects the presence of antibodies or antigens that specifically bind with the labeled antigens or the labeled antibodies.

Fluorescence immunoassay is a biological molecule detecting method that does not employ radioactive substances. Fluorescence immunoassay apparatuses, in which antibodies are immobilized onto a reaction layer in advance (referred to as a solid phase), a measurement target solution and antibodies labeled with fluorescent molecules are caused to flow onto the reaction layer, and fluorescence in the vicinity of the reaction layer is observed to measure the concentration of antigens which have specifically bound to the antibodies, are known (refer to Patent Document 1, for example).

However, fluorescence immunoassay that utilizes solid phases has a problem that it is costly to produce the solid phases. There is a method that utilizes fluorescence polarization method to confirm antigen antibody reactions in solutions (referred to as a liquid phase) as a method that does not employ solid phases. The fluorescence polarization method is a method that detects changes in degrees of fluorescence polarization caused by changes in Brownian motion that occurs by the sizes of molecules changing by molecules binding with molecules which have fluorescent labels. The biological molecule detecting method that utilizes the fluorescence polarization method is known as a simple and expedient method for detecting detection target substances within samples (refer to Patent Document 2, for example).

[Prior Art Documents]

[Patent Documents]

    [Patent Document 1]
    Japanese Unexamined Patent Publication No. 7(1995)-120397
    [Patent Document 2]
    Japanese Unexamined Patent Publication No. 2008-298743

However, the fluorescence polarization method utilizes changes in Brownian motion, which is random, and therefore has a problem that there is a limit to measurement sensitivity. In addition, biological molecule detecting apparatuses that utilize the fluorescence polarization method are not being considered.

The present invention has been developed in view of the foregoing circumstances. It is an object of the present invention to provide a biological molecule detecting apparatus and a biological molecule detecting method which are capable of highly sensitive measurements.

Disclosure of the Invention

A biological molecule detecting apparatus of the present invention that achieves the above object is that which causes a detection target substances, a specific binding substance that specifically binds to the detection target substance, and fluorescent molecules to bind to each other within a solution, and detects fluorescence emitted by the fluorescent molecules to detect or quantify the detection target substance, and has a configuration comprising:

a light source that emits excitation light having a light component which is linearly polarized in a specific direction that excites the fluorescent molecules;

orientation control means for switching the orientation of the fluorescent molecules within the solution to orientations in at least two directions;

a light receiving section that detects the fluorescence emitted by the fluorescent molecules; and

a calculating section that detects or quantifies the detection target substance based on the fluorescence detected by the light receiving section.

In the biological molecule detecting apparatus of the present invention, the orientation control means may switch the orientation of the fluorescent molecules between an orientation in a first direction in which the direction of the transition moments of the fluorescent molecules and the vibration direction of the excitation light are parallel, and an orientation in a second direction in which the direction of the transition moments of the fluorescent molecules and the vibration direction of the excitation light are perpendicular. The "vibration direction" of light refers to the vibration direction of an electric field, and is the same as the polarization direction of light in the case that the light is polarized.

The biological molecule detecting apparatus of the present invention mat adopt a configuration, in which:

the orientation control means switches the orientation of the fluorescent molecules at predetermined temporal intervals;

the light receiving section detects fluorescence a plurality of times; and

the calculating section calculates an arithmetic mean of a plurality of detected fluorescent intensities, and detects or quantifies the detection target substance based on the arithmetic mean of the fluorescent intensities.

In this case, it is preferable for the predetermined temporal intervals to be determined based on the mass or the volume of the detection target substance, the specific binding substances, and the fluorescent molecules, and the degree of orientation control exerted by the orientation control means.

In the biological molecule detecting apparatus of the present invention, it is preferable for the orientation control means to control the orientation of the fluorescent molecules by emitting light having a wavelength different from that of the excitation light. In this case, it is preferable for the orientation control means to be that which emits light having a wavelength different from that of the excitation light onto the solution from a plurality of positions. It is also preferable for the solution to be held in a solution holding portion having a flat surface at least at a portion thereof.

Further, in the case that the solution holding portion has a flat surface, it is preferable for the orientation means to be that which emits light having a wavelength different from that of the excitation light in a direction that passes through the solution and exits the flat surface of the solution holding portion such that the light is conjugately focused with the excitation light emitted by the light source at an interface between the solution and the flat surface.

In the biological molecule detecting apparatus of the present invention, it is preferable for the light receiving section to be equipped with spectral means for spectrally separating light. In this case, it is preferable for the spectral means to be a plurality of filters having different properties, and for the light receiving section to switch a filter to be employed from among the plurality of filters according to the light emitting wavelength of the fluorescent molecules.

In the biological molecule detecting apparatus of the present invention, it is preferable for the calculating section to detect or to quantify the detection target substance by utilizing the fact that there are differences in the temporal changes in the intensity of fluorescence emitted by the fluorescent molecules which are bound to the detection target substance via the specific binding substance and the temporal changes in the intensity of fluorescence emitted by the fluorescent molecules which are not bound to the detection target substance during the switch of the orientation by the orientation control means from a first direction to a second direction.

A biological molecule detecting method of the present invention is a method for causing a detection target substance, a specific binding substance that specifically binds to the detection target substance, and fluorescent molecules to bind to each other within a solution, and detects fluorescence emitted by the fluorescent molecules to detect or quantify the detection target substance, and comprises the steps of:

emitting excitation light having a light component which is linearly polarized in a specific direction that excites the fluorescent molecules;

switching the orientation of the fluorescent molecules within the solution to orientations in at least two directions;

detecting the fluorescence emitted by the fluorescent molecules; and

detecting or quantifying the detection target substance based on the detected fluorescence.

The present invention enables highly sensitive detection of biological molecules.

Brief Description of the Drawings

Figure 1A is a first schematic diagram that illustrates antigen antibody reactions in a biological molecule detecting apparatus according to a first embodiment.

Figure 1B is a second schematic diagram that illustrates antigen antibody reactions in the biological molecule detecting apparatus according to the first embodiment.

Figure 2A is a schematic diagram that illustrates a case in which the vibration direction of excitation light and the transition moment of a fluorescent molecule are parallel.

Figure 2B is a schematic diagram that illustrates a case in which the vibration direction of excitation light and the transition moment of a fluorescent molecule are perpendicular.

Figure 3A is a schematic diagram that illustrates a free molecule.

Figure 3B is a schematic diagram that illustrates a binding molecule.

Figure 4A is a perspective view that illustrates the outer appearance of the biological molecule detecting apparatus according to the first embodiment.

Figure 4B is a diagram that illustrates the biological molecule detecting apparatus according to the first embodiment in a state in which an openable portion is opened.

Figure 5 is a block diagram that illustrates the main components of the biological molecule detecting apparatus.

Figure 6 is a schematic diagram that illustrates switching of the emission direction of laser beams emitted by an orientation controlling light source.

Figure 7A is a schematic diagram that illustrates the relationship between a first laser emission direction and the orientation direction of a molecule.

Figure 7B is a schematic diagram that illustrates the relationship between a second laser emission direction and the orientation direction of a molecule.

Figure 8A is a schematic diagram that illustrates the relationship between a first laser emission direction and the orientation directions of a plurality of molecules.

Figure 8B is a schematic diagram that illustrates the relationship between a second laser emission direction and the orientation directions of a plurality of molecules.

Figure 9 is a schematic diagram that illustrates the detailed structure of a light receiving section of the biological molecule detecting apparatus according to the first embodiment.

Figure 10 is a diagram that schematically illustrates the flow of a process from preparation of a sample through disposal thereof.

Figure 11 is a collection of graphs that illustrate an orientation control signal, a sampling clock, PD output, and A/D converting section output for a single cycle in the biological molecule detecting apparatus according to the first embodiment.

Figure 12 is a graph that illustrates orientation control signals for a plurality of cycles in the biological molecule detecting apparatus according to the first embodiment.

Figure 13A is a first schematic diagram that illustrates antigen antibody reactions in a biological molecule detecting apparatus according to a second embodiment.

Figure 13B is a second schematic diagram that illustrates antigen antibody reactions in the biological molecule detecting apparatus according to the second embodiment.

Figure 14 is a block diagram that illustrates the main components of the biological molecule detecting apparatus according to the second embodiment.

Figure 15 is a schematic diagram that illustrates the detailed structure of a light receiving section of the biological molecule detecting apparatus according to the second embodiment.

Figure 16A is a first graph that illustrates the output from an A/D converting section of the biological molecule detecting apparatus according to the second embodiment.

Figure 16B is a second graph that illustrates the output from the A/D converting section of the biological molecule detecting apparatus according to the second embodiment.

Figure 17A is a conceptual diagram that illustrates the relationship between the transition moment of a fluorescent molecule and the vibration direction of randomly polarized excitation light in the case that a laser beam is emitted from a first direction.

Figure 17B is a conceptual diagram that illustrates the relationship between the transition moment of a fluorescent molecule and the vibration direction of randomly polarized excitation light in the case that a laser beam is emitted from a second direction.

Figure 18A is a conceptual diagram that illustrates the relationship between the transition moment of a fluorescent molecule and the vibration direction of excitation light which is linearly polarized in two directions in the case that a laser beam is emitted from a first direction.

Figure 18B is a conceptual diagram that illustrates the relationship between the transition moment of a fluorescent molecule and the vibration direction of excitation light which is linearly polarized in two directions in the case that a laser beam is emitted from a second direction.

Figure 19 is a collection of graphs that illustrate the changes in the output of photodiodes accompanying changes in the direction of the transition moment of a fluorescent molecule.

Figure 20A is a first conceptual diagram for explaining changes in the orientation of a binding molecule accompanying changes in the polarization axis of a laser beam.

Figure 20B is a second conceptual diagram for explaining changes in the orientation of a binding molecule accompanying changes in the polarization axis of a laser bream.

Figure 20C is a third conceptual diagram for explaining changes in the orientation of a binding molecule accompanying changes in the polarization axis of a laser beam.

Figure 21 is a conceptual diagram that illustrates a case in which linearly polarized laser beams are emitted onto a plurality of points in a reagent cup from the bottom surface thereof.

Figure 22 is a conceptual diagram that illustrates the structure of an orientation controlling light source for casing linearly polarized laser beams to be emitted onto a plurality of points from a predetermined direction.

Figure 23 is a conceptual diagram that illustrates an example of the structure of an optical system for casing linearly polarized laser beams to be emitted onto a plurality of points from a predetermined direction.

Figure 24 is a conceptual diagram that illustrates another example of the structure of an optical system for casing linearly polarized laser beams to be emitted onto a plurality of points from a predetermined direction.

Figure 25 is a conceptual diagram that illustrates a microlens array.

Figure 26 is a conceptual diagram that illustrates an example of the shape of a reagent cup.

Figure 27 is a conceptual diagram that illustrates an example of the positional relationship between the focal point of a focused laser beam and a reagent cup.

Best Mode for Carrying Out the Invention Hereinafter, embodiments of the present invention will be described with reference to the attached drawings. Various specific reactions are utilized to detect biological molecules. Here, apparatuses that utilize specific reactions between antigens and antibodies, and detect antigens which have reacted with the antibodies, based on fluorescence emitted by fluorescent molecules which are bound to the antibodies as labels, will be described as examples.

(First Embodiment)

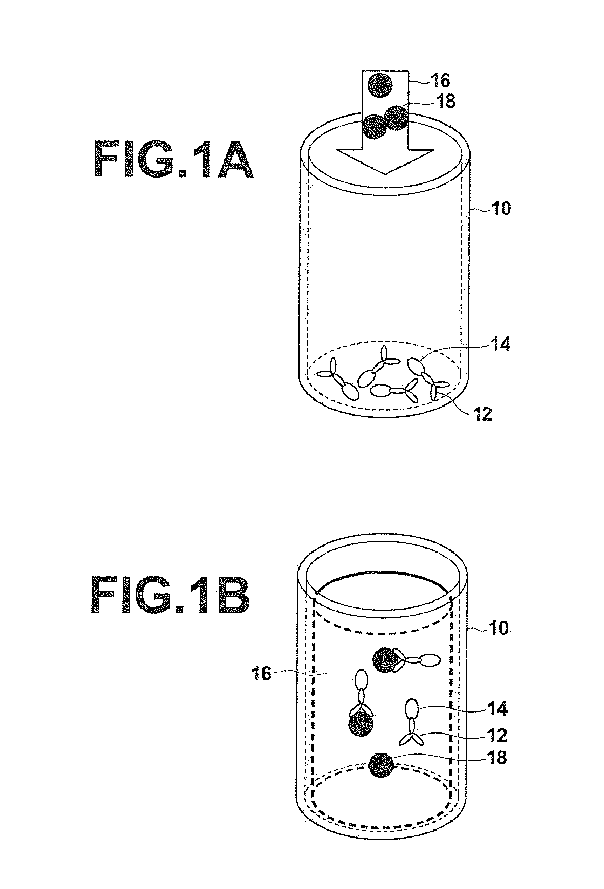

Figure 1A and Figure 1B are schematic diagrams that illustrate antigen antibody reactions in a biological molecule detecting apparatus according to a first embodiment. Antigen antibody reactions within a liquid will be described with reference to Figure 1A and Figure 1B. Here, a case will be considered in which antibodies 12 labeled with fluorescent molecules 14 are contained in a cylindrical reagent cup 10. Plasma separated from whole blood is the sample from which biological molecules are detected. The plasma 16 is dispensed into the reagent cup 10 and stirred. In the case that antigens 18 that specifically bind with the antibodies 12 are present in the plasma 16, antigen antibody reactions will occur between the antibodies 12 and the antigens 18, and the antibodies 12 and the antigens 12 will be present within the plasma 16 in a specifically bound state, as illustrated in Figure 1B. A sufficiently great amount of the antibodies 12 is supplied with respect to the antigens 18. However, there are cases in which the entire amounts of the antibodies 12 and antigens 18 do not undergo antigen antibody reactions and a portion of the antibodies 12 or the antigens 18 remain within the plasma 16 without undergoing antigen antibody reactions. Hereinafter, the antibodies 12, the antigens 18, and the fluorescent molecules 14 which are bound to each other by antigen antibody reactions will be referred to as binding molecules, and the antigens 12 and the fluorescent molecules 14 which have not undergone antigen antibody reactions but are present in the liquid will be referred to as free molecules. The binding molecules and the free molecules are both present in the plasma 16. Note that components other than the antigens 18 are present in the plasma 16. However, components other than the antigens 18 are omitted from Figure 1A and Figure 1B in order to simplify the description.

The biological molecule detecting apparatus according to the first embodiment of the present invention emits excitation light into the solution in which the binding molecules and the free molecules are both present. Fluorescence emitted by the fluorescent molecules 14 is received, and detection or quantification of the antigens 18 is performed based on fluorescence data obtained thereby. Accordingly, it is desirable for only fluorescence emitted from the binding molecules that include the antigens 18 to be detected. However, the free molecules and the binding molecules are both present in the solution. Therefore, when the excitation light is emitted into the solution, the fluorescent molecules 14 associated with the free molecules also emit fluorescence, resulting in unnecessary fluorescent components being generated. Therefore, the biological molecule detecting apparatus according to the first embodiment of the present invention calculates the fluorescence contributed by fluorescent molecules associated with free molecules from among the entirety of the fluorescence data.

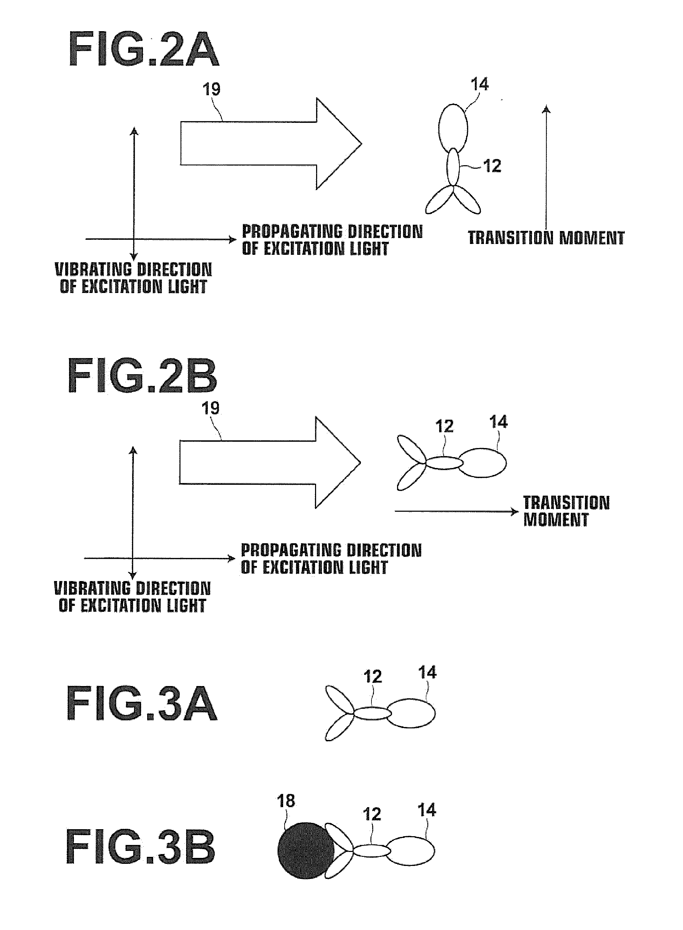

Excitation efficiency of the fluorescent molecules 14 by linearly polarized excitation light will be described with reference to Figures 2A and 2B in order to explain the principle of calculating the fluorescence contributed by the binding molecules and the fluorescence contributed by the free molecules in the biological molecule detecting apparatus 100 according to the first embodiment. Figure 2A is a schematic diagram that illustrates a case in which the vibration direction of excitation light 19 and the transition moment of a fluorescent molecule 14 are parallel. Figure 2B is a schematic diagram that illustrates a case in which the vibration direction of excitation light 19 and the transition moment of a fluorescent molecule 14 are perpendicular. Here, cases are described in which the longitudinal direction of the fluorescent molecule 14 is parallel to the orientation direction of the transition moment, to simplify the description.

The fluorescent molecules 14 transition to an excited state when light energy is absorbed, and emits fluorescence during the process of returning to a baseline state. When a fluorescent molecule 14 is excited by linearly polarized excitation light 19, the fluorescent molecule 14 emits fluorescence which is polarized in the same direction as the polarization direction of the excitation light. The degree of polarization of fluorescence emitted by the fluorescent molecules 14 depends on the speed of rotational movement thereof. That is, if a fluorescent molecule 14 is not undergoing rotational movement, the fluorescent molecule 14 emits fluorescence which is polarized in the same direction as the vibration direction of the excitation light 19. The degree of polarization of the fluorescence emitted by fluorescent molecules 14 decreases as the speed at which they are undergoing rotational movement becomes greater. When the fluorescent molecules 14 are excited, vectors within the fluorescent molecules called transition moments, which are determined by the molecular structures of the fluorescent molecules 14, interact with the excitation light 19. The transition moments have unique directions within the fluorescent molecules 14, and the relationship between the directions of the transition moments and the vibration direction of the excitation light 19 determines the excitation efficiency of the fluorescent molecules 14. Specifically, the fluorescent molecules 14 selectively absorb light that vibrates in a direction parallel to the transition moments thereof. Accordingly, in the case that the excitation light 19 is emitted onto a fluorescent molecule 14 while vibrating in the vertical direction of the drawing sheet and propagating from the left to the right of the drawing sheet as illustrated in Figures 2A and 2B, the excitation efficiency becomes greatest in the case that the vibration direction of the excitation light 19 is parallel to the transition moment of the fluorescent molecule 14 (Figure 2A), and becomes 0 in the case that the vibration direction of the excitation light 19 is perpendicular to the transition moment of the fluorescent molecule 14 (Figure 2B). The orientations of the transition moments change according to the orientations of the fluorescent molecules 14, and therefore the orientations of the fluorescent molecules 14 within the solution influence the excitation efficiencies thereof.

Motion of the free molecules and the binding molecules within the solution will be described with reference to Figures 3A and 3B in order to consider the orientations of the fluorescent molecules 14 within the solution. Figure 3A is a schematic diagram that illustrates an antibody 12 and a fluorescent molecule 14, the combination of which is a free molecule. Figure 3B is a schematic diagram that illustrates an antibody 12, an antigen 18, and a fluorescent molecule 14, the combination of which is a binding molecule. The free molecules and the binding molecules move irregularly (Brownian motion) within the solution, and undergo movement within the solution and rotational movement. It is known that Brownian motion of molecules within solutions is influenced by absolute temperature, the volumes of the molecules, the viscosity of the solutions, etc. The volumes of the binding molecules are greater than those of the free molecules due to the antigens 18 being bound thereto, and are less likely to undergo Brownian motion within the solution. A technique (fluorescence polarization immunoassay) that utilizes the difference in Brownian motion of free molecules and binding molecules within solutions to detect binding molecules from changes in Brownian motion is known. However, because Brownian motion is random, the great number of binding molecules and free molecules which are present in the solution are in randomly oriented states, and signals which are the sums of the signals output therefrom are detected. Therefore, the S/N ratio is poor, and detection sensitivity is limited.

The biological molecule detecting apparatus according to the first embodiment of the present invention utilizes laser beams to control the orientations of molecules within the solution. The free molecules and the binding molecules are separated based on the degree of tracking with respect to the control, to realize high sensitivity. When a laser beam is emitted onto the free molecules and the binding molecules within the solution, the free molecules and the binding molecules which had been moving randomly within the solution become oriented in a specific direction (hereinafter, a state in which the molecules are oriented in a specific direction by receiving external force will be referred to as "complete molecular orientation"). Because the ease with which the free molecules and the binding molecules move and rotate within the solution differ, in the case that the orientations of the molecules within the solution are controlled by the laser beam, the amount of time required for the free molecules and the binding molecules to complete molecular orientation from the time that the laser beam is emitted differ. The biological molecule detecting apparatus according to the first embodiment of the present invention utilizes the difference in the amounts of time required for the free molecules and the binding molecules to complete molecular orientation to generate differences in the excitation efficiencies of the fluorescent molecules 14 associated with each type of molecule and to calculate the fluorescence contributed by the binding molecules.

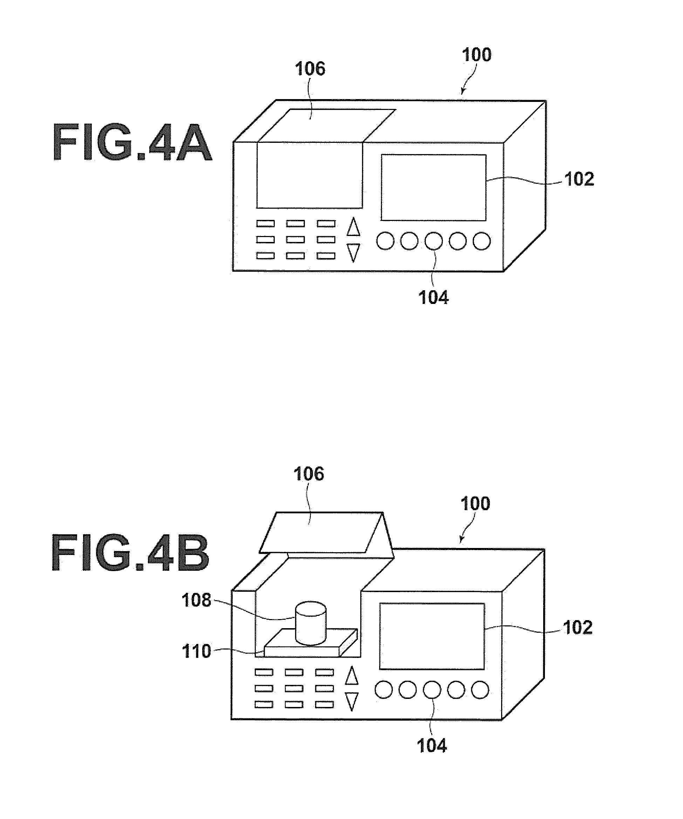

Next, the configuration of the biological molecule detecting apparatus 100 according to the first embodiment of the present invention will be described. Figure 4A is a perspective view that illustrates the outer appearance of the biological molecule detecting apparatus 100. A display section 102, an operating section 104, and an openable portion 106 are provided on a side surface of the biological molecule detecting apparatus 100. The display section 102 displays measurement results and the like. The operating section 104 is a section at which modes are set, sample data are input, etc. The openable portion 106 is of a configuration in which an upper lid may be opened. The upper lid is opened when samples are set, and closed during measurements. By adopting this configuration, light from the exterior influencing measurements can be prevented.

Figure 4B is a perspective view that illustrates the biological molecule detecting apparatus 100 in a state in which the openable portion 106 is opened. When the openable portion 106 is opened, a reagent cup 108 and a holding base 110 are present within the biological molecule detecting apparatus 100. The reagent cup 108 is removably held by the holding base 110. The reagent cup 108 is a cylindrical container in which solutions are placed. Users dispense samples into the reagent cup 108 and close the upper lid to perform measurements. Although not illustrated in the drawings, the biological molecule detecting apparatus 100 is also equipped with a reagent tank and a dispensing section. When measurements are initiated, the dispensing section suctions a reagent from within the reagent tank, and dispenses the reagent into the reagent cup 108.

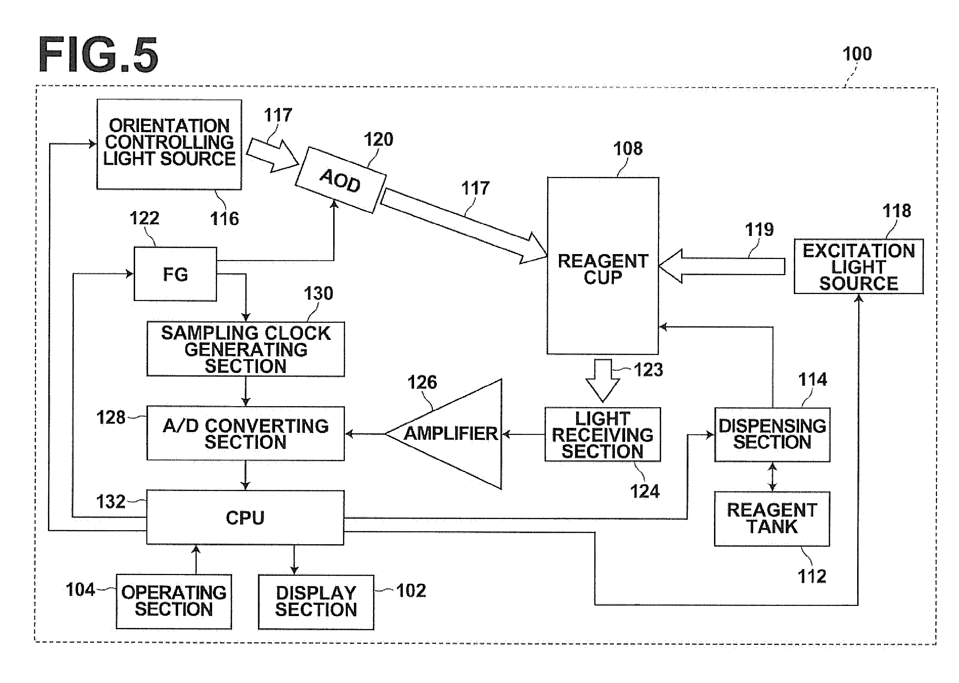

Figure 5 is a functional block diagram that illustrates the main components of the biological molecule detecting apparatus 100. The reagent tank 112 and the dispensing section 114 are provided within the biological molecule detecting apparatus 100. The reagent tank 112 is a tank having a plurality of types of reagents stored therein. The dispensing section 114 suctions reagents to be utilized from the reagent tank 112 with a pipette, then dispenses the reagent into the reagent cup. In addition, an orientation controlling light source 116 and an excitation light source 118 are provided within the biological molecule detecting apparatus 100. The orientation controlling light source 116 emits an orientation controlling laser beam 117 towards an AOD (Acoustic Optic Deflector) 120, to control the orientations of molecules within the solution in the reagent cup 108 by applying external force.

The AOD 120 utilizes the acoustic optical effect to change the refractive index of the interior thereof based on input voltages, to switch the direction in which light input thereto propagates. That is, the AOD 120 changes the refractive index of the interior thereof to change the direction in which the laser beam 117 propagates, based on voltages input according to voltage signals (hereinafter, the signals output to the AOD 120 will be referred to as "orientation control signals") output from a FG (Function Generator) 122. In other words, the direction in which the laser beam 117 propagates is determined by the orientation control signals generated by the FG 122.

The FG 122 is a device capable of generating voltage signals having various frequencies and waveforms. The FG 122 outputs different voltage signals to the AOD 120 and a sampling clock generating section 130 in response to commands received from a CPU 132.

The CPU 132 specifies orientation control signals to be output by the FG 122, to control the timings at which the AOD 120 switches the direction in which the laser beams 117 propagates.

The excitation light source 118 emits excitation light 119, which is linearly polarized by a polarizing element provided within the excitation light source 118, that excites the fluorescent molecules 14, toward the reagent cup 108.

A light receiving section 124 is provided under the reagent cup 108. The light receiving section 124 receives fluorescence 123 generated by the fluorescent molecules 14 within the reagent cup 108 under the reagent cup 108, converts the received fluorescence signals to analog electrical signals (analog fluorescence data), and outputs the analog electrical signals to an amplifier 126.

The amplifier 126 amplifies the analog fluorescence data output thereto from the light receiving section 124, and outputs the amplified analog fluorescence data to an A/D converting section 128.

The sampling clock generating section 130 inputs a sampling clock that specifies the timings at which the A/D converting section 128 is to sample the analog fluorescence data to the A/D converting section, based on voltage signals output thereto from the FG 122.

The A/D converting section 128 samples the analog fluorescence data output thereto from the amplifier 126, based on the sampling clock output thereto from the sampling clock generating section 130. The A/D converting section 128 converts the sampled analog fluorescence data to digital data, and outputs the digital data to the CPU 132.

The CPU 132 performs calculations using the digital data output thereto from the A/D converting section 128, and outputs the results of calculations to the display section 102. In addition, the CPU 132 controls the operations of the orientation controlling light source 116, the excitation light source 118, the dispensing section 114, and the FG 122 in response to commands input from the operating section 104. Specifically, the CPU 132 outputs ON/OFF commands to the orientation controlling light source 116 and the excitation light source 118, outputs commands that specify a reagent to be utilized and commands to initiate dispensing operations to the dispensing section 114, and outputs commands that specify the waveform of voltage signals to be output and commands to output the voltage signals to the FG 122.

In the first embodiment, a laser having a wavelength of 1.3μm and an output of 700mW is employed as the orientation controlling light source 116, and a light source having a wavelength of 532nm and an output of 10mW is employed as the excitation light source 118.

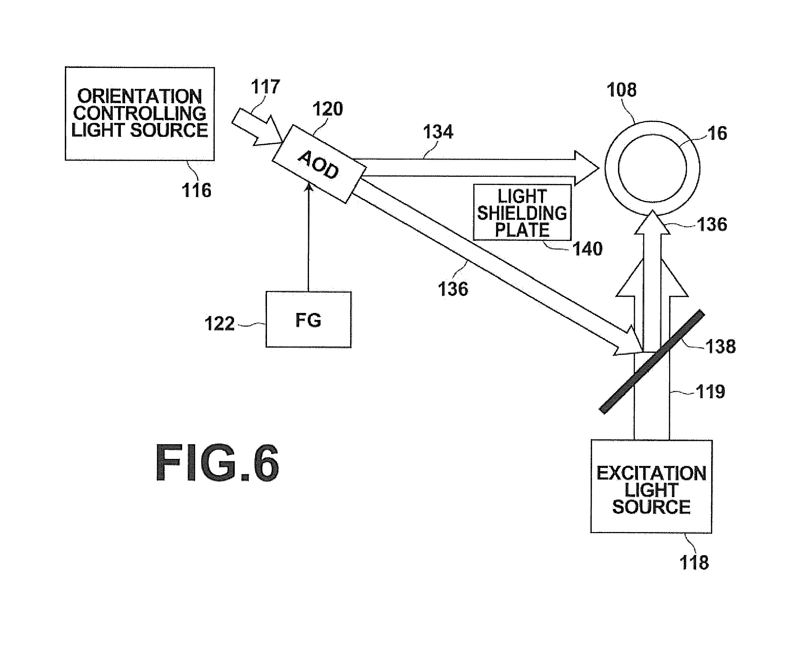

Figure 6 is a schematic plan view of the interior of the biological molecule detecting apparatus 110 that illustrates switching of the emission direction of the laser beam emitted by the orientation controlling light source 116. The switching of the emission direction of the laser beam with respect to the reagent cup 108 will be described with reference to Figure 6. The laser beam 117 emitted from the orientation controlling light source 116 passes through the AOD 120 and enters the reagent cup 108. The laser beam 117 emitted by the orientation controlling light source 116 is of a width that enables the entirety of the solution within the reagent 108 to be irradiated thereby. The AOD 120 alternately switches the direction that the laser beam emitted from the orientation controlling light source 116 propagates between two directions. Specifically, the AOD 120 causes the laser beam 117 to propagate in the direction of laser beam 134 in the case that a 5V orientation control signal is input thereto, and causes the laser beam 117 to propagate in the direction of laser beam 136 in the case that a 0V orientation control signal is input thereto. The laser beam 134 enters the side surface of the reagent cup 108. The laser beam 136 is reflected by a dichroic mirror 138, propagates in a direction perpendicular to the laser beam 134, and enters the side surface of the reagent cup 108. If the reagent cup 108 viewed from above is considered as a clock face, the laser beam 134 enters from the 9 o'clock position and propagates toward the 3 o'clock position, whereas the laser beam 136 enters from the 6 o'clock position and propagates toward the 12 o'clock position. That is, the directions in which the laser beam 134 and the laser beam 136 propagate are perpendicular to each other. The dichroic mirror 138 only reflects light having the wavelength of the laser beam 117, and transmits light having other wavelengths. The excitation light 119 emitted from the excitation light source 118 passes through the dichroic mirror 138, propagates in the same direction as the laser beam 136 reflected by the dichroic mirror 138, and enters the side surface of the reagent cup 108.

This configuration enables the biological molecule detecting apparatus 100 to alternately switch the direction in which the laser beam enters the reagent cup 108 between two directions which are 90 degrees different from each other, by controlling the AOD 120 according to the input of the orientation control signals from the FG 122. A light shielding plate 140 is placed between the AOD 120 and the reagent cup 108, and the biological molecule detecting apparatus 100 is configured such that laser beams that propagate in directions other than those of the laser beam 134 and the laser beam 136 do not enter the reagent cup 108. In addition, the laser beam enters the side surface of the cylindrical reagent cup 108 both in the case that it propagates in the direction of the laser beam 134 and in the direction of the laser beam 136. Because the reagent cup 108 is cylindrical, the shape of the side surface of the reagent cup 108 that the laser beam enters is the same even if the direction that the laser propagates in is switched.

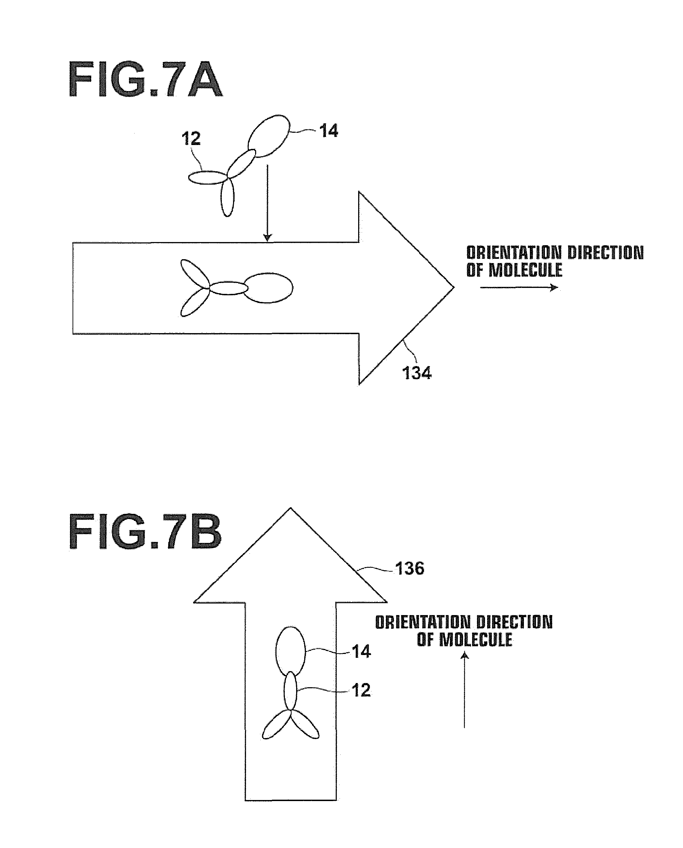

The motion of fluorescent molecules within the reagent cup 108 in response to the switching of the emission direction of the laser beam will be described with reference to Figures 7A and 7B. Figure 7A is a schematic diagram that illustrates the relationship between a first laser emission direction and the orientation direction of a molecule. Figure 7B is a schematic diagram that illustrates the relationship between a second laser emission direction and the orientation direction of a molecule. Note that in the present specification, the "orientation direction" of the free molecules and the binding molecules refer to a direction in which the antibodies and the fluorescent molecules are oriented after orientation thereof is completed. The free molecules and the binding molecules are dispersed within the solution oriented in random directions. However, the free molecules and the binding molecules within the reagent cup 108 into which the laser beam is emitted become oriented in a specific direction by receiving the external force of the laser beam. The external force exerted by the laser is generated by reactions to the laser beam hitting the free molecules and binding molecules and being scattered. The direction in which the force is exerted is determined by the direction in which the laser beam is propagating and the orientations of the free molecules and binding molecules. Here, a description will be given with respect to a free molecule as an example. As illustrated in figure 7A, a free molecule on which the laser beam 134 is emitted receives force in a rotational direction from the laser beam 134, and stabilizes in an orientation in a direction in which the external force in the rotational direction imparted by the laser balances out (here, the same direction as the direction in which the laser beam 134 propagates). In other words, the free molecule receives external force to rotate in the rightward or leftward direction in cases that the free molecule is not oriented in the direction in which the laser beam 134 propagates. However, in the case that the free molecule is oriented in the direction in which the laser beam 134 propagates, the external rotational force in the rightward or leftward direction balance out, and therefore the free molecule stabilizes while oriented in a single direction. Meanwhile, the antibody 12 and the fluorescent molecule 14, on which the laser beam 136 is emitted, stabilizes while oriented in a direction perpendicular to the direction into which the laser beam 134 effects orientation. By changing the emission direction of the laser beam in this manner, it is possible to switch the directions in which the antibodies 12 and fluorescent molecules 14 within the solution are oriented.

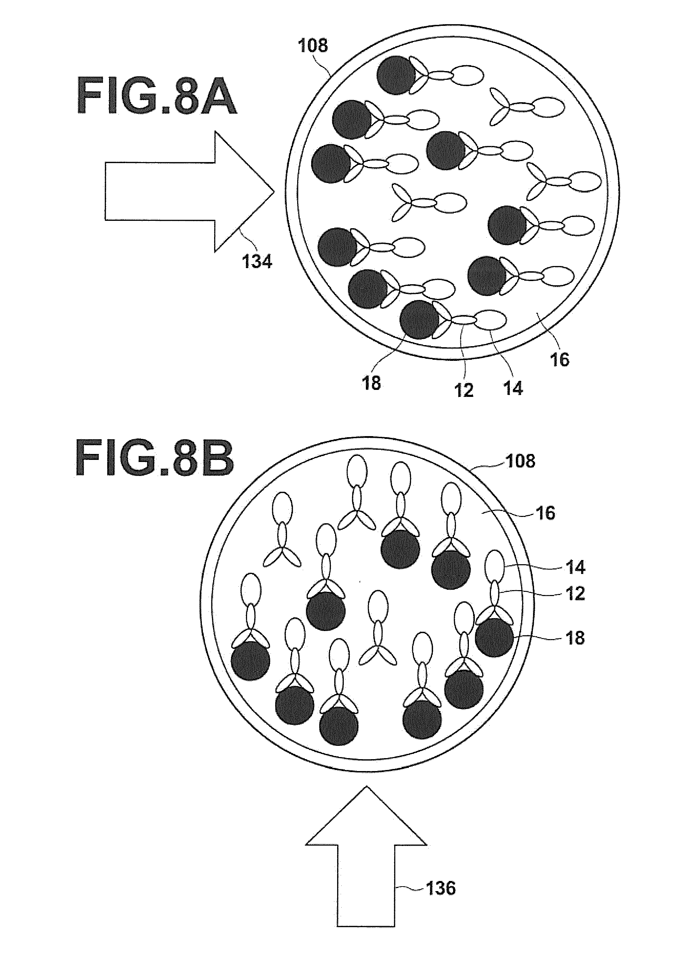

Figures 8A and 8B are schematic diagrams that illustrate the relationships between laser emission directions and the orientation directions of a plurality of molecules. Figures 8A and 8B are views of the reagent cup 108 from above. Figure 8A illustrates a case in which free molecules and binding molecules are oriented in the direction that the laser beam 134 propagates due to being irradiated by the laser beam 134, which is propagating from the left of the drawing sheet to the right of the drawing sheet. The free molecules and the binding molecules within the solution that receives irradiation of the laser beam 134 are oriented in the same direction. That is, the transition moments of all of the fluorescent molecules 14 are aligned in a single direction. Figure 8B illustrates a case in which free molecules and binding molecules are oriented in the direction that the laser beam 136 propagates due to being irradiated by the laser beam 136, which is propagating from the bottom of the drawing sheet to the top of the drawing sheet. When the laser beam 134 is switched to the laser beam 136, the free molecules and the binding molecules which were oriented toward the right side of the drawing sheet receive forces to rotate in the leftward direction. In this case, the free molecules, which have smaller volumes than the binding molecules, rotate faster than the binding molecules, and stabilize in orientations in the direction that the laser beam 136 propagates. That is, if a sufficient amount of time elapses, the free molecules and the binding molecules will be oriented in the same direction. However, the amounts of time required for the two types of molecules to complete orientation differ. In the case that the laser beam 136 is emitted as well, if all of the free molecules and binding molecules complete orientation, the transition moments of all of the fluorescent molecules 14 will be aligned in a single direction.

In the first embodiment, the directions of the transition moments of the fluorescent molecules 14 which have been oriented by the laser beam 134 are parallel to the direction in which the linearly polarized excitation light vibrates, maximizing the excitation efficiency of the fluorescent molecules 14. Meanwhile, the directions of the transition moments of the fluorescent molecules 14 which have been oriented by the laser beam 136 are perpendicular to the direction in which the linearly polarized excitation light vibrates, and the excitation efficiency of the fluorescent molecules 14 is 0. Accordingly, switching of the emission direction of the laser beam by the AOD 120 switches the excitation efficiency of the fluorescent molecules 14 with respect to the linearly polarized excitation light between a maximum and a minimum (in which excitation does not occur).

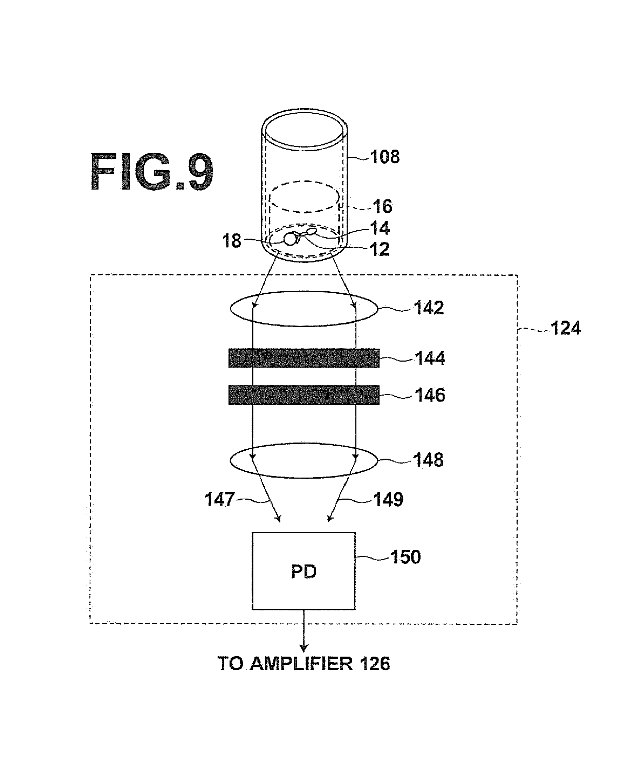

Next, the detailed structure of the light receiving section 124 will be described with reference to Figure 9. Figure 9 is a schematic diagram that illustrates the detailed structure of the light receiving section 124. The light receiving section 124 includes: a lens 142; a filter 144; a polarizing element 146; a lens 148; and a PD (photodiode) 150. The light receiving section 124 receives fluorescence from the bottom side of the reagent cup 108. The fluorescence 123 emitted by the fluorescent molecules 14 within the reagent cup 108 is focused by the lens 142, and enters the PD 150 after passing through the filter 144, the polarizing element 146 and the lens 148. In Figure 9, the width of the fluorescence 123 is illustrated using arrow 147 and arrow 149. The filter 144 is a band pass filter that cuts off light other than the fluorescence emitted by the fluorescent molecules 14, and prevents light other than the fluorescence, such as the excitation light, from entering the PD 150. The polarizing element 146 only transmits light which is polarized in the same direction as the vibration direction of the linearly polarized excitation light. The excitation light which is scattered within the reagent cup 108 and fluorescence emitted by the fluorescent molecules 14 while the directions of the orientations of the free molecules and the binding molecules are being switched have vibration directions different from the original vibration direction of the excitation light, and therefore cannot be transmitted through the polarizing element 146. The PD 150 receives the fluorescence which is focused by the lens 148, generates electrical charges corresponding to the intensity of the fluorescence, and outputs the generated electrical charges to the amplifier 126. The light receiving section 124 converts the fluorescence emitted by the fluorescent molecules 14 of which the orientations have been switched into electrical charges in this manner. In addition, the light receiving section 124 receives the fluorescence toward the bottom side of the reagent cup 108. Therefore, the light receiving section 124 is not likely to be influenced by the laser beam 117 and the excitation light 119.

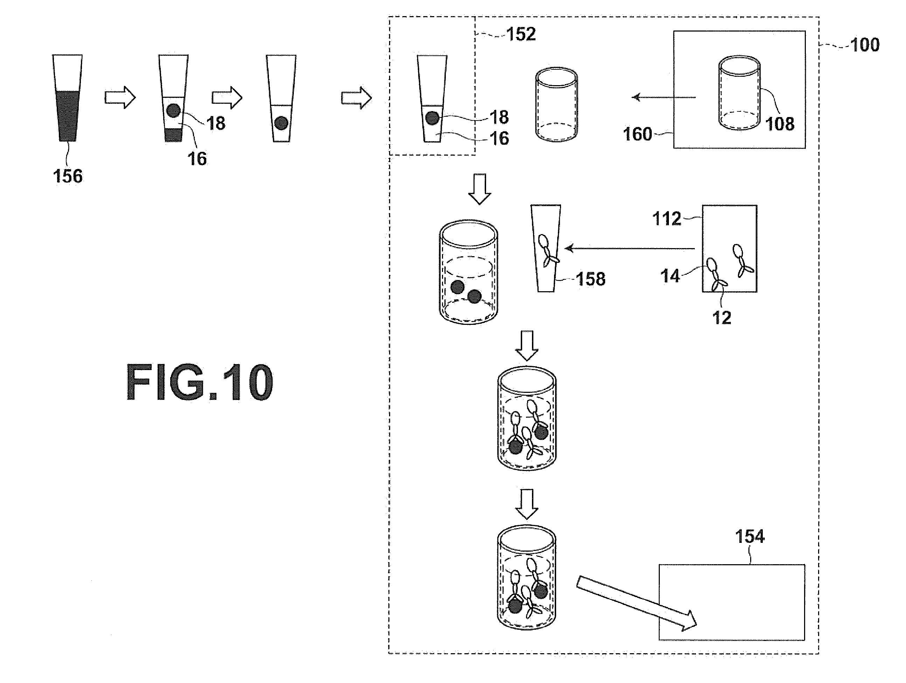

Next, the operations of the biological molecule detecting apparatus 100 during measurements will be described. Figure 10 is a diagram that schematically illustrates the flow of a process from preparation of a sample through disposal thereof. In the first embodiment, a case will be considered in which whole blood is employed as a sample, P53 (Protein 53) is a detection target substance as the antigens 18, and P53 antibodies is a substance that specifically binds with the detection target substance as the antibodies 12. Alexa Fluor 555 by Molecular Probes is employed as the fluorescent molecules 14. Alexa Fluor 555 emits fluorescence having wavelengths within a range from 550nm to 700nm, with a peak at approximately 570nm. In the case that Alexa Fluor 555 is utilized as the fluorescent molecules 14, a light receiving side filter of a SpOr-A filter set by Semrock is employed. This filter is a band pass filter that transmits wavelengths within a range from 575nm to 600nm, and transmits a portion of the fluorescence emitted by Alexa Fluor 555. The capacity of the reagent cup 108 is approximately 120μL.

To prepare for measurement, first, 50μL of whole blood 156 collected from a patient is centrifugally separated to separate plasma 16. The separated plasma 16 is set in a sample setting section of the biological molecule detecting apparatus 100. The steps up to this point are performed by a user. The biological molecule detecting apparatus 100 dispenses the plasma 16, which is set in the sample setting section 152, into a new reagent cup 108, which is stocked in a reagent cup stocking section 160. Next, the biological molecule detecting apparatus 100 suctions P53 antibodies, which are in the reagent tank 112, with a pipette 158, and dispenses the suctioned P53 antibodies into the reagent cup 108. The biological molecule detecting apparatus 100 which has placed the plasma 16 and the P53 antibodies into the reagent cup 108 uses a built in vortex mixer to agitate the reagent cup 108 while maintaining the temperature of the reagent cup 108 at 37°C to cause antigen antibody reactions to occur. Thereafter, the biological molecule detecting apparatus 100 emits excitation light, detects fluorescence, and disposes of the reagent cup 108 into a built in trash receptacle 154 after the fluorescence is detected.

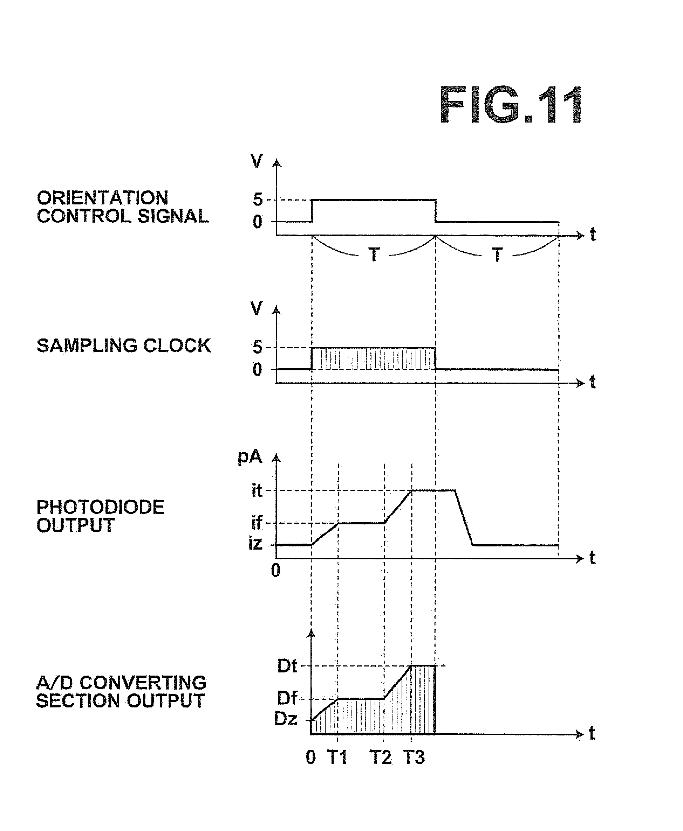

The orientation control signals output by the FG 122, the sampling clock output by the sampling clock generating section 130, the PD outputs output by the PD 150, and the A/D conversion outputs output by the A/D converting section will be described with reference to Figure 11. Figure 11 is a collection of graphs having orientation control signals, sampling clocks, PD outputs, and A/D converting section outputs as vertical axes, respectively, and time t as the horizontal axes for a single cycle in the biological molecule detecting apparatus 100. Note that here, the graphs of the PD outputs and the A/D converting section outputs are illustrated schematically in order to simplify the description.

The orientation control signal output by the FG 122 is 0V prior to measurement. In the case that the orientation control signal is 0V, the sampling clock is also 0V, and sampling is not performed. The A/D converting section output is also 0 because the sampling clock is not input. The value of noise iz caused by the apparatus is output as an initial PD output. The reason why only noise is output from the PD 150 is because the laser beam 117 emitted by the orientation controlling light source 116 propagates in the direction of the laser beam 136 by the 0V orientation control signal being input to the AOD 120, the transition moments of all of the fluorescent molecules 14 within the solution are oriented in a direction perpendicular to the vibration direction of the excitation light 119, and it is not possible for the excitation light 119 to excite the fluorescent molecules 14.

Next, the biological molecule detecting apparatus 100 changes the orientation control signal to 5V, and emits the excitation light 119 toward the reagent cup 108. When the orientation control signal is changed to 5V, the sampling clock generating section 130 periodically outputs a 5V signal as a sampling clock that represents sampling timings. The A/D converting section 128 samples analog fluorescence data at timings that match the sampling clock and performs A/D conversion. In addition, when the orientation control signal is changes to 5V, the AOD 120 switches the direction in which the laser beam 117 propagates from the direction of the laser beam 136 to that of the laser beam 134. Accompanying the switching of the direction in which the laser beam 117 propagates, the direction, in which the laser beam is emitted onto the reagent cup 108 is switched by 90 degrees. Therefore, the directions in which the fluorescent molecules 14 within the reagent cup 108 are oriented are also switched by 90 degrees.

Accompanying the switch in the emission direction of the laser beam, the directions of orientations of the free molecules, which have smaller volumes than the binding molecules, switch first, resulting in fluorescence being emitted by the fluorescent molecules thereof due to the transition moments thereof becoming not perpendicular to the vibration direction of the excitation light. A majority of the fluorescence emitted by the fluorescent molecules 14 associated with the free molecules during the switch in orientations thereof is not polarized, and therefore is cut off by the polarizing element 146. The excitation efficiency of the free molecules which have completed being reoriented are maximal, because the transition moments of the fluorescent molecules thereof are parallel to the vibration direction of the excitation light. The fluorescence emitted by the fluorescent molecules associated with the free molecules, of which reorientation is complete, reach the PD 150 without being cut off by the polarizing element 146, because it is polarized in the same direction as the vibration direction of the excitation light. Accompanying the increase in the free molecules for which reorientation has been completed, the PD output increases from iz. When reorientation of all of the free molecules is completed at time T1, the PD output is temporarily saturated at value if. Thereafter, when reorientation of the binding molecules begins at time T2, the PD output increases again. A majority of the fluorescence emitted by the fluorescent molecules 14 associated with the binding molecules during the switch in orientations thereof is not polarized, and therefore is cut off by the polarizing element 146. When reorientation of all of the binding molecules is completed at time T3, the PD output is saturated at value it.

The output of the A/D converting section is initially a value Dz, similar to the PD output, then gradually increases and becomes temporarily saturated at a value Df. The output of the A/D converting section gradually increases accompanying the increase in the PD output from if, and becomes saturated at a value Dt. By setting the orientation control signal to be 0V and then changing it to 5V in this manner, the fluorescence contributed by the fluorescent molecules associated with the free molecules and the fluorescence contributed by the fluorescent molecules associated with the binding molecules will appear in the output of the A/D converting section with a temporal difference therebetween.

The orientation control signal is changed back to 0V after being output as 5V for T seconds. T seconds is set to be greater than or equal to the amount of time required for the output of the PD to become saturated a second time at the value it. By the orientation control signal being switched back to 0V from 5V, the sampling clock will also become 0V. After the orientation control signal is switched from 5V to 0V, the PD output will be the value it for an amount of time, and then decrease to the value iz. The reason why the PD output becomes the value iz that represents only noise caused by the apparatus is because the transition moments of the fluorescent molecules 14 become oriented in a direction perpendicular to the vibration direction of the excitation light due to the orientation control signal being switched to 0V, and the excitation efficiency of the fluorescent molecules 14 becomes 0, resulting in fluorescence not being emitted. The reason why the PD output is the value it for an amount of time is because the switching of the orientations of the fluorescent molecules 14 is slightly delayed from the switching of the orientation control signal. Here, the period of time during which the orientation control signal is set at 0V is T seconds, which is the same amount of time that the orientation control signal was set at 5V. This is because the amount of time required for the free molecules and the binding molecules within the solution to complete being reoriented is approximately the same for a case that the orientation control signal is switched from 0V to 5V and a case that the orientation control signal is switched from 5V to 0V, under conditions that the laser output is constant. A single measurement cycle of the biological molecule detecting apparatus 100 is from the point in time that the orientation control signal is switched from 0V to 5V to a point in time T seconds after orientation control signal is switched back to 0V. That is, the amount of time in a single measurement cycle of the biological molecule detecting apparatus 100 is 2T seconds.

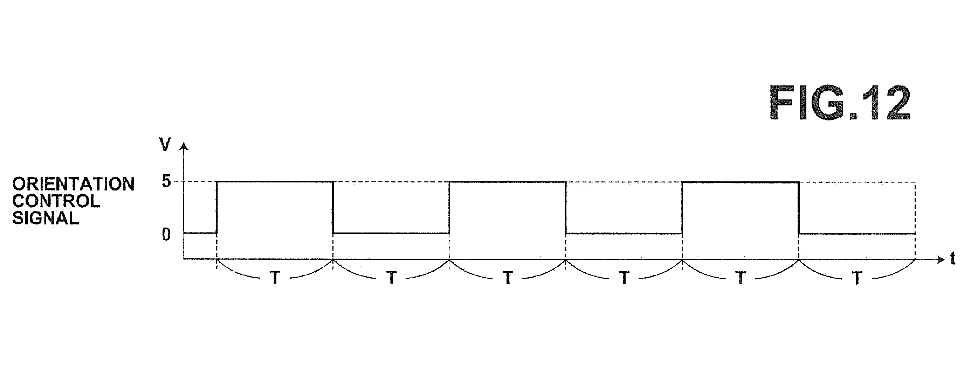

Figure 12 is a graph that illustrates orientation control signals for a plurality of cycles in the biological molecule detecting apparatus 100. As illustrated in Figure 12, the biological molecule detecting apparatus 100 performs a plurality of measurement cycles by switching the orientation control signals at predetermined temporal intervals, calculates arithmetic means for each of a plurality of values Dt, Df, and Dz, and obtains the average values for Dt, Df, and Dz. In the first embodiment, 10 measurement cycles are performed to obtain the average values for Dt, Df, and Dz. Thereby, fluctuations in measurement results caused by a variety of factors can be averaged out.

The CPU 132 calculates the concentration of the binding molecules from the obtained average values for Dt, Df, and Dz. Specifically, first, a measurement value S is calculated according to Formula (1) below.

$$S=(Dt-Df)/(Dt-Dz) \qquad\qquad (1)$$

In Formula (1), (Dt-Df) represents the intensity of fluorescence emitted by the fluorescent molecules associated with the binding molecules. (Dt-Dz) represents the intensity of combined fluorescence emitted by the binding molecules and the free molecules, and is calculated by subtracting data related to apparatus noise from the maximum obtained data value. Factors that deteriorate the reproducibility of measurement results, such as changes in optical systems, are canceled by dividing (Dt-Df) by (Dt-Dz).

The CPU 132 calculates a diagnostic value C (the concentration of the detection target substance) from the obtained measurement value S. The diagnostic value C is calculated according to Formula (2) below.

$$C=f(S) \qquad\qquad (2)$$

Here, f(S) is a calibration curve function. The biological molecule detecting apparatus 100 has different calibration curve functions for each item to be measured prepared in advance, and converts the measurement value S to the diagnostic value C. The CPU 132 outputs the obtained diagnostic value C to the display section 102.

As described above, the biological molecule detecting apparatus 100 according to the first embodiment of the present invention is of a configuration that switches the emission direction of the laser beam, thereby enabling switching of the orientation directions of the free molecules and the binding molecules within the solution. The directions in which the free molecules and the binding molecules are oriented by the laser beam are that in which the transition moments of the fluorescent molecules of the free molecules and the binding molecules are parallel to the vibration direction of the linearly polarized excitation light, and that in which the transition moments of the fluorescent molecules of the free molecules and the binding molecules are perpendicular to the vibration direction of the linearly polarized excitation light. That is, the biological molecule detecting apparatus 100 is capable of switching between a state in which the fluorescent molecules of the free molecules and the binding molecules are capable of being excited by the excitation light and a state in which the fluorescent molecules of the free molecules and the binding molecules not capable of being excited by the excitation light. In addition, there is a difference in the amounts of time required for reorientation of the free molecules and the binding molecules to become complete accompanying switching of the emission direction of the laser beam. Therefore, the timings at which fluorescence emitted by the fluorescent molecules associated with each type of molecule is received differ. Accordingly, the biological molecule detecting apparatus 100 can calculate the fluorescence contributed by the fluorescent molecules associated with the free molecules and the fluorescence contributed by the fluorescent molecules associated with the binding molecules separately, and the concentration of the detection target substance can be accurately measured with a simple structure.

In the configuration described above, the biological molecule detecting apparatus 100 switches the orientations of all of the free molecules and the binding molecules into the same direction by the external force exerted by the laser beam. Therefore, measurements having higher sensitivity can be performed compared to cases in which measurements are performed utilizing Brownian motion, which is random.

Note that the first embodiment was described as a case in which antigen antibody reactions are utilized as an example. However, the combination of the detection target substance and the substance that specifically binds with the detection target substance is not limited to the case described above. For example, the present invention may be applied to cases in which antigens are employed to detect antibodies, cases in which a specific nucleic acid is employed to detect a nucleic acid that hybridizes with the specific nucleic acid, cases in which nucleic acids are employed to detect nucleic acid binding proteins, cases in which ligands are employed to detect receptors, cases in which sugars are employed to detect lectin, cases in which protease detection is utilized, cases in which higher order structure changes are utilized, etc.

In the first embodiment, calculations were described for a case in which the graph illustrating measurement results is a schematic graph, to simplify the description of the calculation of the concentration of the detection target substance from the measurement results. However, it is not necessary for calculations to be performed in the manner described above. For example, a boundary point between fluorescence emitted by free molecules and fluorescence emitted by binding molecules may be determined based on the inflection point within a graph, to perform calculations.

In addition, it is desirable for the period of time during which the orientation control signal is set to 5V or 0V to be changed, based on the volumes of the free molecules and the binding molecules, the viscosity of the solution, the temperature of the solution, etc. The amount of time required for reorientation of the molecules to become complete following switching of the emission direction of the laser beam is determined by the ease with which the free molecules and the binding molecules rotate within the solution, which is influenced by the volumes of the free molecules and the binding molecules, the viscosity of the solution, the temperature of the solution, etc. In cases that it is difficult for the free molecules and the binding molecules to rotate within the solution, the amount of time required for reorientation of the free molecules and the binding molecules to be completed will become longer. Therefore, it is desirable for the period of time during which the orientation control signal is set to 5V or 0V to be long enough for reorientation to be completed. In this case, it is not necessary for the period during which the orientation control signal is set to 5V and the period during which the orientation control signal is set to 0V to be the same amount of time.

In addition, the first embodiment employed a laser light source that emits a laser beam having a wavelength of $1.3\mu m$ and an output of 700mW as the orientation controlling light source 116. However, the orientation controlling light source 116 is not limited to such a laser light source. It is desirable for the wavelength and the output of the laser light source to be determined based on the ease with which the free molecules and the binding molecules rotate within the solution, which is influenced by the volumes of the free molecules and the binding molecules, the masses of the free molecules and the binding molecules, etc. Particularly, it is desirable for a laser having an output to a degree that results in a difference to be exhibited in the amounts of time required for reorientation of the free molecules and the binding molecules to become complete.

Note that the first embodiment was described as a case in which measurements were repeatedly performed and arithmetic means of the measurement results were obtained. However, calculation of the arithmetic mean is not necessary, and the calculations may be determined according to what is important to a user. For example, in the case that a user wishes to perform measurements expediently, measurement may be performed for only a single cycle and the results of the measurement maybe displayed. Alternatively, in the case that a user wishes to perform measurements with higher precision, measurements may be repeated for a plurality of cycles, to improve the measurement accuracy.

(Second Embodiment)

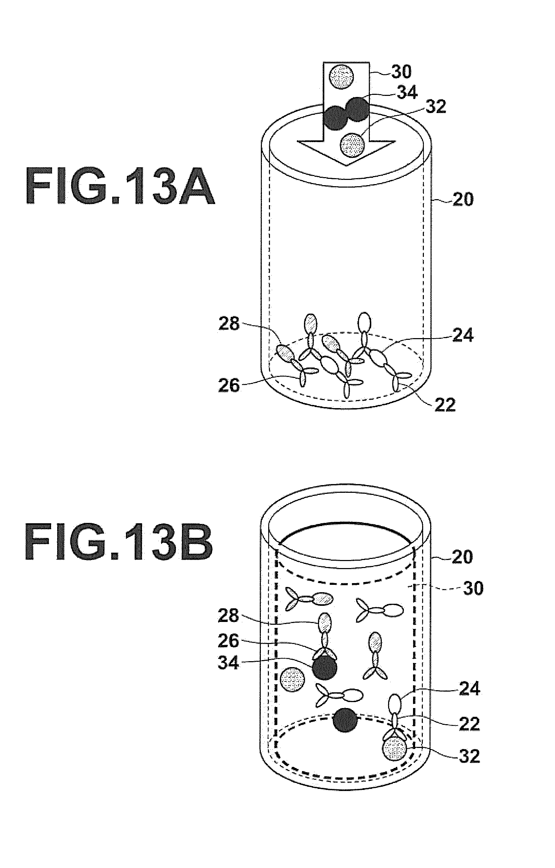

Figures 13A and 13B are schematic diagrams that illustrate antigen antibody reactions in a biological molecule detecting apparatus according to a second embodiment. The second embodiment utilizes two types of antibodies to detect two types of antigens. Hereinafter, a case will be considered in which antibodies 22 and antibodies 26 are placed within a reagent cup 20.

The antibodies 22 and the antibodies 26 are labeled with fluorescent molecules 24 and fluorescent molecules 28, respectively. When a sample 30 is placed in the reagent cup 20 and agitated, and if antigens 32 that specifically bind with the antibodies 22 are present in the in the sample 30, antigen antibody reactions will occur between the antibodies 22 and the antigens 32. Similarly, if antigens 34 that specifically bind with the antibodies 26 are present in the in the sample 30, antigen antibody reactions will occur between the antibodies 26 and the antigens 34. In the same manner as that described with respect to the first embodiment, a portion of the antibodies and the antigens remain within the sample solution without undergoing antigen antibody reactions. Hereinafter, the antibodies 22, the antigens 32, and the fluorescent molecules 24 which are bound to each other by antigen antibody reactions will be referred to as binding molecules 1, and the antigens 22 and the fluorescent molecules 24 which have not undergone antigen antibody reactions but are present in the liquid will be referred to as free molecules 1. Further, the antibodies 26, the antigens 34, and the fluorescent molecules 28 which are bound to each other by antigen antibody reactions will be referred to as binding molecules 2, and the antigens 26 and the fluorescent molecules 28 which have not undergone antigen antibody reactions but are present in the liquid will be referred to as free molecules 2. In the second embodiment, the antigens 32 and the antigens 34, which are detection target substances, are P53 and CEA (Carcinoembryonic Antigen), respectively. P53 antibodies that specifically bind to P53 are employed as the antibodies 22, and CEA antibodies that specifically bind to CEA are employed as the antibodies 26. Alexa Fluor 555 by Molecular Probes is employed as the fluorescent molecules 24, and Alexa Fluor 588 by Molecular Probes is employed as the fluorescent molecules 28. Alexa Fluor 588 emits fluorescence having wavelengths within a range from 575nm to 750nm, with a peak at approximately 610nm.

The biological molecule detecting apparatus according to the second embodiment of the present invention emits excitation light onto the solution, in which the two types of free molecules and the two types of binding molecules are present, and detects or quantifies target binding molecules.

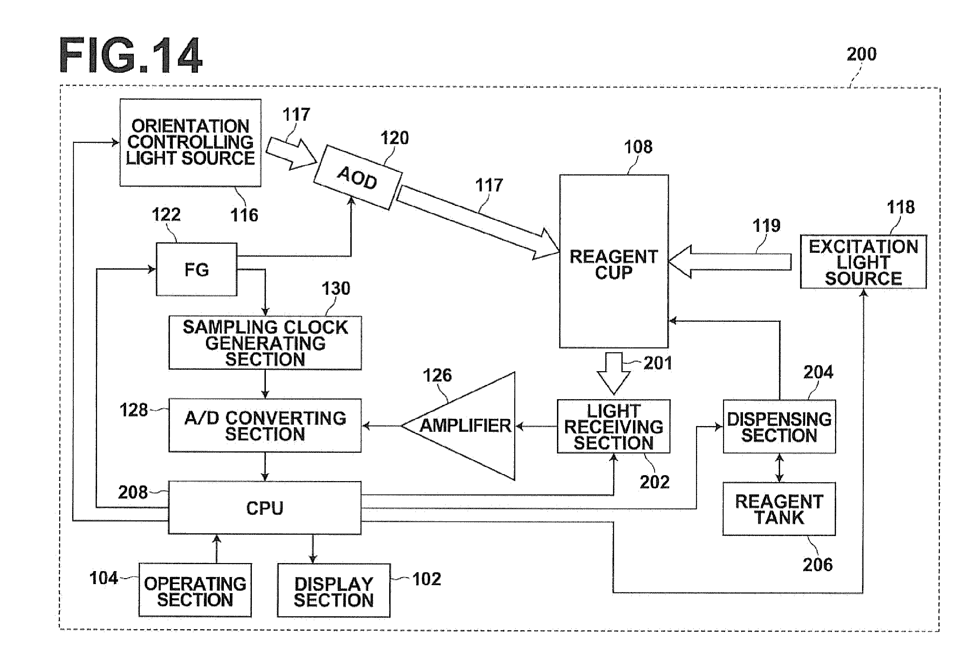

Figure 14 is a block diagram that illustrates the main components of the biological molecule detecting apparatus 200 according to the second embodiment. Note that constituent elements of the biological molecule detecting apparatus 200 which are the same as those of the biological molecule detecting apparatus 100 of the first embodiment are denoted with the same reference numerals, and detailed descriptions thereof will be omitted. The biological molecule detecting apparatus 200 is different from the biological molecule detecting apparatus 100 of the first embodiment in the configurations of a light receiving section 202, a dispensing section 204, a reagent tank 206, and a CPU 208.

The dispensing section 204 suctions two types of antibodies from the reagent tank 206, which stores a plurality of antibodies in separate containers, and dispenses the suctioned antibodies into the reagent cup 108.

The light receiving section 202 detects fluorescence emitted by the fluorescent molecules within the reagent cup 108. The light receiving section 202 is configured to receive fluorescence emitted by the fluorescent molecules 24 and fluorescence emitted by the fluorescent molecules 28 separately in response to commands from the CPU 208.

The CPU 208 performs calculations on digital data output thereto from the A/D converting section 128, and outputs the results of calculation to the display section 102. In addition, the CPU 208 controls the operations of the orientation controlling light source 116, the excitation light source 118, the dispensing section 204, the FG 122, and the light receiving section 202 in response to commands input from the operating section 104. Specifically, the CPU 132 outputs ON/OFF commands to the orientation controlling light source 116 and the excitation light source 118, outputs commands that specify reagents to be utilized and commands to initiate dispensing operations to the dispensing section 204, outputs commands that specify the waveform of voltage signals to be output and commands to output the voltage signals to the FG 122, and outputs commands to switch filters to the light receiving section 202.

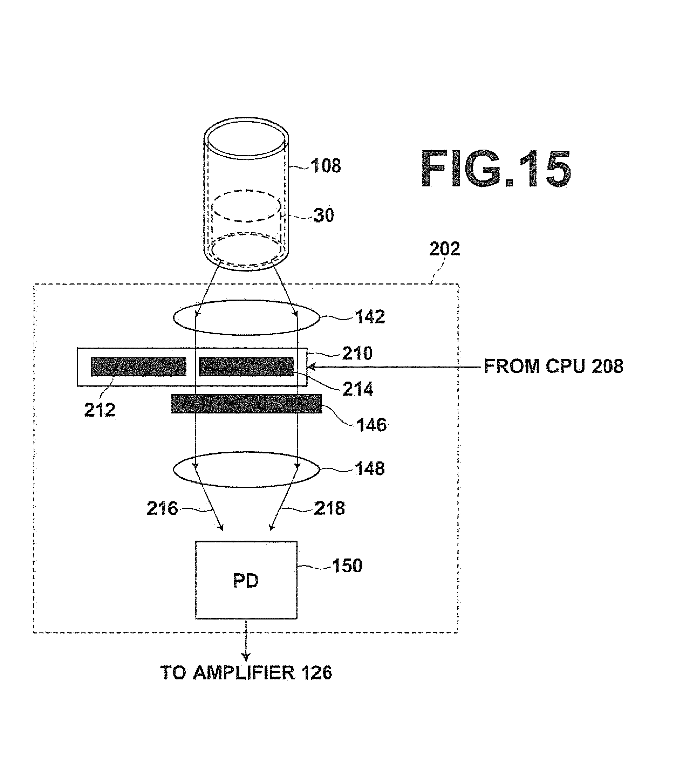

The configuration of the light receiving section 202 will be described in detail with reference to Figure 15. Figure 15 is a schematic diagram that illustrates the detailed structure of the light receiving section 202 of the biological molecule detecting apparatus 200 according to the second embodiment. A filter switching section 210 within the light receiving section 202 is equipped with two types of filters, a filter 212 and a filter 214. The two filters are movable, and the filter switching section 210 is configured to enable switching of the filter through which light focused by the lens 142 passes. The filter switching section 210 switches the filter to be utilized in response to commands output thereto from the CPU 208. For example, the filter 212 is utilized when detecting fluorescence emitted by Alexa Fluor 555, and the filter 214 is utilized when detecting fluorescence emitted by Alexa Fluor 588. Thereby, unnecessary fluorescence is prevented from reaching the PD 150. In the second embodiment, a light receiving side filter of a SpOr-A filter set by Semrock is employed as the filter 212, and a light receiving side filter of a SpRed-A filter set by Semrock is employed as the filter 214. The light receiving side filter of the SpRed-A filter set is a band pass filter that transmits wavelengths within a range from 605nm to 650nm. Note that unnecessary fluorescence is prevented from reaching the PD 150 by spectrally separating the fluorescence using the two types of filters. However, it is not necessary to spectrally separate light using filters. For example, only light having specific wavelengths may be received by spectrally separating light using a diffraction grating or a prism.

Next, the operations of the biological molecule detecting apparatus 200 during measurements will be described. The measurement operations of the biological molecule detecting apparatus 200 are basically the same as the measurement operations of the biological molecule detecting apparatus 100 of the first embodiment, but differ in fine points. The principle behind detecting the free molecules and the binding molecules separately was described with respect to the first embodiment, and therefore how the two types of binding molecules are detected separately will be described here. First, the biological molecule detecting apparatus 200 determines which of the two types of binding molecules are to be detected. This determination may be performed as desired, by user input via the operating section 104, for example. Here, a case will be described in which the binding molecules 1 having Alexa Fluor 555 as fluorescent molecules are detected first will be described. The CPU 208 outputs a command that instructs the filter switching section 210 within the light receiving section 202 to utilize the filter 212. The filter switching section 210 receives the command from the CPU 208, and moves the filter 212 to a position at which light focused by the lens 142 passes. When the orientation control signal is changed to 5V and excitation light is emitted toward the reagent cup 108, fluorescence is emitted by the fluorescent molecules 24 and the fluorescent molecules 28 within the solution. The fluorescence emitted by the fluorescent molecules 24 and the fluorescent molecules 28 is focused by the lens 142 and enters the filter 212. The filter 212 only transmits light having wavelengths within a range from 575nm to 600nm. Therefore, the fluorescence emitted by the fluorescence molecules 24 passes through the filter 212, while the fluorescence emitted by the fluorescent molecules 28 is substantially completely shielded. Only the fluorescence emitted by the fluorescent molecules 24 can be detected in this manner.

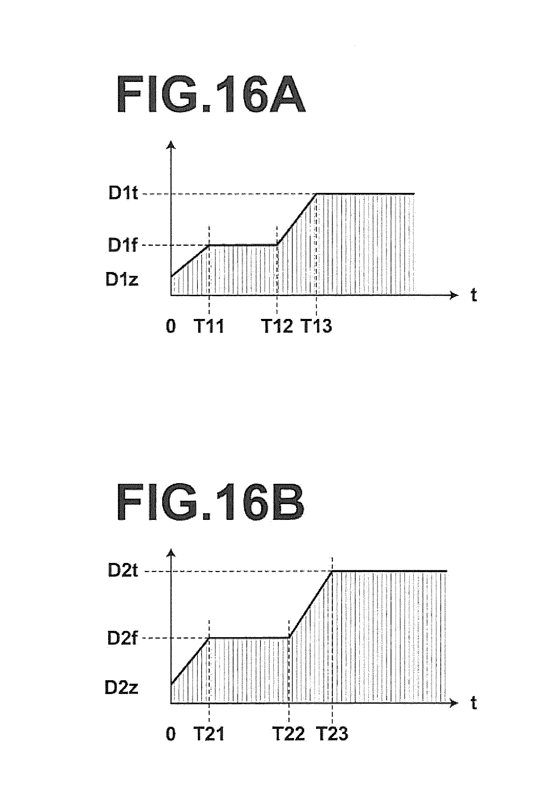

The output of the A/D converting section resulting from performing a single measurement cycle with the biological molecule detecting apparatus 200 detecting the fluorescence emitted by the fluorescent molecules 24 is illustrated in Figure 16A. Note that here, the graph of Figure 16A is illustrated schematically in order to simplify calculations. The A/D converting section outputs a value D1z that represents apparatus noise, which gradually increases and becomes temporarily saturated at time T11 at a value D1f. Thereafter, the output of the A/D converting section begins to increase again at time T12, and becomes saturated again at time T13 at a value D1t.

The biological molecule detecting apparatus 200 switches the orientation control signal at predetermined temporal intervals to perform a plurality of cycles of measurements, calculates arithmetic means for a plurality of D1t values, D1f values, and D1z values, and obtains average values for each of the D1t values, D1f values, and D1z values.

Next, the CPU 208 calculates the concentration of the binding molecules 1 from the obtained average values for D1t, D1f, and D1z. Specifically, the same calculation as that performed when obtaining the measurement value S in the first embodiment are performed to obtain a measurement value S1. Then, a calibration curve function f1(S) is employed to convert the measurement value S1 to a concentration C1. The CPU 208 outputs the obtained concentration C1 to the display section 102.

Next, the biological molecule detecting apparatus 200 performs measurement of the binding molecules 2. The CPU 208 outputs a command that instructs the filter switching section 210 within the light receiving section 202 to utilize the filter 214. The filter switching section 210 receives the command from the CPU 208, and moves the filter 214 to a position at which light focused by the lens 142 passes. The filter 214 only transmits light having wavelengths within a range from 610nm to 650nm. Therefore, the fluorescence emitted by the fluorescence molecules 24 is shielded by the filter 214, while the fluorescence emitted by the fluorescent molecules 28 is transmitted therethrough. Only the fluorescence emitted by the fluorescent molecules 28 can be detected in this manner.

The output of the A/D converting section resulting from performing a single measurement cycle with the biological molecule detecting apparatus 200 detecting the fluorescence emitted by the fluorescent molecules 28 is illustrated in Figure 16B. Note that here, the graph of Figure 16B is illustrated schematically in order to simplify calculations. The A/D converting section outputs a value D2z that represents apparatus noise, which gradually increases and becomes temporarily saturated at time T21 at a value D2f. Thereafter, the output of the A/D converting section begins to increase again at time T22, and becomes saturated again at time T23 at a value D2t.

The biological molecule detecting apparatus 200 switches the orientation control signal at predetermined temporal intervals to perform a plurality of cycles of measurements, calculates arithmetic means for a plurality of D2t values, D2f values, and D2z values, and obtains average values for each of the D2t values, D2f values, and D2z values.

The timings at which the orientation control signals are switched when the binding molecules 2 are measured are different from those when the binding molecules 1 are measured. This is because the volumes and masses of the binding molecules 1, the free molecules 1, the binding molecules 2, and the free molecules 2 are different, and the amounts of time required for orientation of the molecules to be completed differ.

As illustrated in Figures 16A and 16B, the timings at which the PD outputs increase and become saturated differ between the case that the binding molecules 1 are measured and the case that the binding molecules 2 are measured. This difference is due to the difference in the ease with which the binding molecules 1 and the binding molecules 2 move within the solution, caused by the difference in the volumes thereof.

Next, the CPU 208 calculates the concentration of the binding molecules 2 from the obtained average values for D2t, D2f, and D2z. Specifically, the same calculation as that performed when obtaining the measurement value S in the first embodiment are performed to obtain a measurement value S2. Then, a calibration curve function f2(S) is employed to convert the measurement value S2 to a concentration C2. The CPU 208 outputs the obtained concentration C2 to the display section 102.

As described above, the biological molecule detecting apparatus 200 according to the second embodiment of the present invention employs two types of antibodies and fluorescent molecules as substances that specifically bind with detection target substances and is equipped with the filter switching 210 which enables switching between two types of filters, in addition to having the structures of the biological molecule detecting apparatus 100 of the first embodiment. Thereby, only fluorescence emitted by the fluorescent molecules associated with the binding molecules that include a detection target substance can be detected, by utilizing a filter corresponding to the fluorescent molecules associated with the binding molecules that include the detection target substance. Accordingly, the concentrations of two types of detection target substances which are included in a single sample can be accurately measured.

Simultaneously measuring a plurality of detection target substances from a single sample is important from the viewpoint of improving diagnostic accuracy. For example, if diagnosis is administered using both P53 and CEA, which were detected in the second embodiment, diagnoses having approximately twice the positive rates compared to those administered when these substances are detected singly can be administered with respect to breast cancer.

Note that Alexa Fluor 555 and Alexa Fluor 588 were employed as the fluorescent molecules in the second embodiment. However, the fluorescent molecules are not limited to these. A plurality of substances that specifically bind to a plurality of detection target substances respectively may be labeled with a plurality of types of fluorescent molecules having fluorescent wavelengths which are sufficiently different to be capable of being separated by filters.

Note that the second embodiment was described as a case in which antigen antibody reactions are utilized as an example. However, the combination of the detection target substance and the substance that specifically binds with the detection target substance is not limited to the case described above. For example, the present invention may be applied to cases in which antigens are employed to detect antibodies, cases in which a specific nucleic acid is employed to detect a nucleic acid that hybridizes with the specific nucleic acid, cases in which nucleic acids are employed to detect nucleic acid binding proteins, cases in which ligands are employed to detect receptors, cases in which sugars are employed to detect lectin, cases in which protease detection is utilized, cases in which higher order structure changes are utilized, etc.

In addition, the second embodiment was described as a case in which two types of detection target substances are employed. However, the number of detection target substances is not limited to two. In such cases as well, each of the detection target substances can be detected separately, by employing a plurality of substances that specifically bind with each of the plurality of detection target substances, labeling each of the plurality of specific binding substances with a different type of fluorescent molecules, and detecting the fluorescence emitted by each type of fluorescent molecule by separating the fluorescence with a plurality of filters corresponding to each type of fluorescent molecule.

Note that the number of types of fluorescent molecules will increase as the number of types of detection target substances increases, and fluorescence emitted by the plurality of types of fluorescent molecules will be present, and there may be cases in which it is difficult to separate the fluorescence using only filters. In such cases, the types of excitation light may be increased to facilitate separate the fluorescence. The degrees of light absorption of fluorescent molecules depend on the wavelength of excitation light, and each type of fluorescent molecule has a wavelength band which is easily absorbed. For this reason, changing the wavelength of the excitation light causes only a portion of the fluorescent molecules to emit fluorescence, facilitating separation of the fluorescence using filters. In addition, detection of fluorescence emitted by target fluorescent molecules can be facilitated by employing band pass filters having narrower passbands.

(Design Modifications to the First and the Second Embodiments)

Note that the embodiments of the present invention described above are merely examples of the present invention, and do not limit the structure of the present invention. The biological molecule detecting apparatus of the present invention is not limited to the embodiments described above, and various changes and modifications are possible as long as they do not stray from the objective of the present invention.

For example, the external force applied to the molecules within the solution is not limited to that applied by laser beams. Magnetic methods or electric methods may be employed as long as they apply external force to a degree that causes differences in the amounts of time required for the reorientation of free molecules and binding molecules to become complete.

In addition, in the embodiments described above, the directions in which the laser beam propagates were switched between two directions perpendicular to each other, that is, that which orients the transition moments of the fluorescent molecules associated with the free molecules and the binding molecules to be parallel to the vibration direction of the excitation light, and that which orients the direction of the transition moments of the fluorescent molecules associated with the free molecules and the binding molecules to be perpendicular to the vibration direction of the excitation light. However, it is not necessary for the two directions to be perpendicular to each other. For example, in the case that a detection target substance is to be quantified, it is only necessary for one of the two directions that the laser beam propagates to be that which orients the direction of the transition moments of the fluorescent molecules associated with the free molecules and the binding molecules to be perpendicular to the vibration direction of the excitation light, that is, an orientation that does not enable the linearly polarized excitation light to excite the fluorescent molecules. If the fluorescent molecules are oriented such that the linearly polarized excitation light cannot excite the fluorescent molecules, the PD output will become noise only, because fluorescence will not be emitted. When the emission direction of the laser beam is switched to another direction, only the fluorescence emitted by the fluorescent molecules associated with the free molecules and the binding molecules for which reorientation has been completed can be received. In other words, emission of fluorescence can be temporarily reset, thereby preventing unnecessary fluorescence from being received and eliminating noise due to unnecessary fluorescence. In this case, if the two directions that the laser beam propagates in are perpendicular, the difference in the amounts of time required for reorientation of the free molecules and the binding molecules to be completed becomes maximal, resulting in the highest S/N ratio. Meanwhile, if the angle formed by the two directions that the laser beam propagates in is 60 degrees, the amounts of time required for reorientation of the free molecules and the binding molecules to be completed will be shorter, and the amount of time required to perform measurements will also become shorter. In this manner, as the angle formed by the two directions that the laser beam propagates in becomes closer to 0 degrees, the amounts of time required for reorientation of the free molecules and the binding molecules to be completed will be shorter, and the amount of time required to perform measurements will also become shorter.

In addition, in the case that measurements are performed merely to ascertain whether a detection target substance is present within a solution, that is, whether binding molecules are present, it is only necessary to switch the emission direction of the laser beam into two directions having an angular difference that will cause a difference in the amounts of time required for reorientation of the free molecules and the binding molecules to be completed to occur. That is, it is not necessary for the two directions to include that which orients the direction of the transition moments of the fluorescent molecules associated with the free molecules and the binding molecules to be perpendicular to the vibration direction of the excitation light. If a difference is generated in the amounts of time required for reorientation of the free molecules and the binding molecules to be completed, the difference will be represented in the fluorescence data, and therefore the presence of the binding molecules can be confirmed.

In addition, the AOD 120 was employed to switch the emission direction of the laser beam in the embodiments described above. However, it is not necessary to employ the AOD 120 as long as a structure that enables the laser beam to be emitted in two directions is utilized. For example, a configuration that utilizes a mirror to change the emission direction of the laser beam may be employed. As another alternative, two orientation controlling light sources 116 may be provided to emit laser beams from two directions.

Cases in which a single reagent cup is provided within the biological molecule detecting apparatus were described in the above embodiments. However, it is not necessary for a single reagent cup to be employed, and a configuration may be adopted in which a plurality of reagent cups, in which a plurality of samples are set, are provided in the biological molecule detecting apparatus. In this case, if the apparatus is configured to sequentially move the reagent cups to measurement positions and to perform measurements, a plurality of samples can be automatically measured.

Note that the above embodiments were described as cases in which antibodies labeled with fluorescent molecules were employed. However, it is not necessary to use antibodies which have already been labeled with fluorescent molecules. For example, binding of antibodies and antigens and binding of the antibodies and fluorescent molecules may be simultaneously performed within a reagent cup. In this case, a user may prepare antibodies and fluorescent molecules in separate reagent tanks, and the biological molecule detecting apparatus may dispense the antibodies, the fluorescent molecules, and a sample into a reagent cup, to cause reactions to occur when performing measurements.

In addition, the orientation controlling light source 116 and the excitation light source 118 may be configured to be removable, such that they may be replaced by those appropriate to a detection target substance and the type of fluorescent molecule.

If the detection target substance is detected or quantified by switching the direction in which orientation is controlled by the orientation control means at predetermined temporal intervals, and calculating arithmetic means of the plurality of pieces of obtained fluorescence data, the influence of noise that occurs within each measurement cycle can be reduced, and measurements can be performed with higher accuracy.

It is desirable for the temporal intervals at which the direction of orientation control to be switched to be determined by obtaining the amount of time required for orientation of all free molecules and all binding molecules to be completed, based on the mass or the volume of the detection target substance, the specific binding substance, and the fluorescent molecules, and the degree of orientation control exerted by the orientation control means, and designating the obtained amount of time as the length of the temporal interval. In this case, the laser beam will not be emitted in the same direction after orientation of all of the molecules are completed, thereby reducing power consumption. In addition, measurement will not be continued extraneously, and measurement times can be shortened.

The amount of time required for orientation of all free molecules and all binding molecules to be completed may be obtained based on PD output or the output of the A/D converting section. For example, if several measurement cycles are repeated, the approximate amount of time required for the outputs to become saturated can be understood. Therefore, an arithmetic mean of the amounts of time required for the outputs to become saturated may be calculated, and the calculated amount of time may be designated as the predetermined temporal interval.

Complex mechanisms are obviated in the case that a laser beam is employed to control the orientations of molecules compared to a case that the orientations of molecules are controlled by magnets, etc. In order to control the orientations of molecules using magnets, for example, the molecules need to be magnetic, or magnetic molecules that bind with molecules of which the orientations are to be controlled need to be prepared, and preparations for measurements become complex.

Note that in the embodiments of the present invention described above, cases were described in which whole blood was employed as samples. However, the sample is not limited to being whole blood, and other bodily fluids such as urine and spinal fluid may be employed as samples as long as detection target substances are dispersed within solutions thereof.

In addition, the embodiments of the present invention can perform measurements in a liquid phase, in which antigens, antibodies, and fluorescent molecules are dispersed within a solution, and therefore exhibits the advantage that preliminary processes are simple compared to solid phase measurements.

In addition, the number of orientation controlling light sources is not limited to one for each emission direction in the present invention. A plurality of orientation controlling light sources that emit a plurality of laser beams in a single direction may be provided.

With respect to an optical system that controls the directions of the transition moments of fluorescent molecules by changing the direction in which a laser beam is emitted as in the embodiments of the present invention described above, a plurality of such optical systems may be provided, to simultaneously emit laser beams onto a plurality of points from a certain direction, thereby widening an irradiation range, to avoid a problem that the irradiation range will become small in the case that the beam diameter of the laser beam is decreased. The plurality of optical systems may have a plurality of optical paths at least at a stage prior to the laser beam entering the reagent cup. For example, if three optical systems that also include light sources are provided, laser beams are emitted from all three orientation controlling light sources, and the laser beams can irradiate three points of the reagent cup from a certain direction. As another example, a single laser beam may be branched by employing a two dimensional laser array, a microlens array, etc, the laser beams can be emitted onto a plurality of points corresponding to the number of branches, even if only a single light source is provided. In such cases, laser beams can be simultaneously irradiated onto a plurality of points, and the transition moments of fluorescent molecules can be rotated at a plurality of locations.

The embodiments of the present invention were described as cases in which the excitation light 119, which is linearly polarized in a single direction, is emitted onto the solution. That is, the excitation light 119 has a single polarization plane. However, it is not necessary for the excitation light 119 to be a linearly polarized light beam having a single polarization plane. In order to obtain the same advantageous effects as those obtained by the first embodiment and the second embodiment, the excitation light 119 needs only to have at least one component which is linearly polarized in a specific direction. Here, light which is linearly polarized in a specific direction is light for which changes in the relationship between the transition moments of fluorescent molecules and the vibration direction of the linearly polarized component changes the excitation efficiency of the linearly polarized component with respect to the fluorescent molecules. For example, if randomly polarized excitation light may be emitted and an analyzer may provided in front of the light receiving section such that only components of fluorescence emitted from the fluorescent molecules which are linearly polarized in a specific direction is received. Here, randomly polarized light refers to light in which the vibration direction is random, and a plurality of linearly polarized components that vibrate in various directions are present.

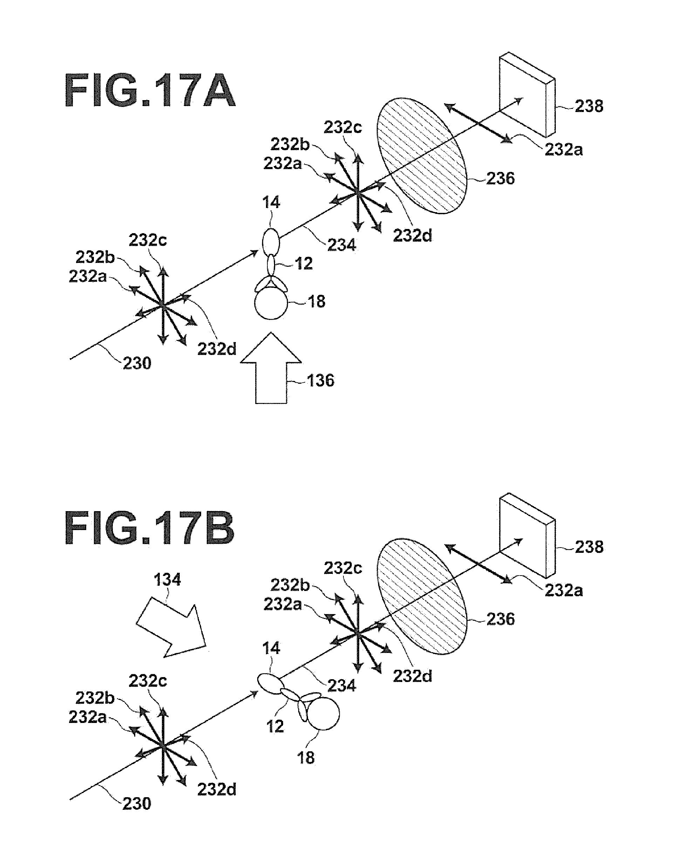

Figures 17A and 17B are conceptual diagrams that illustrate the relationships between the orientation direction of a fluorescent molecule 14 and the vibration directions of randomly polarized excitation light 230 in cases that the laser beam 136 and the laser beam 134 are emitted, respectively. The vibration directions 232a through 232d of the excitation light 230 represent the vibration directions of light within a plane perpendicular to the direction in which the excitation light 230 propagates. In Figure 17A and

Figure 17B, the vibration directions 232a through 232d illustrate that the excitation light 230 vibrates in various directions. In actuality however, many more components having different angular directions are included in addition to the components illustrated in Figure 17A and Figure 17B. When fluorescent molecules which are static within a solution due to being oriented are excited by linearly polarized excitation light, the fluorescent molecules emit fluorescence which is polarized in the same direction as the vibration direction of the excitation light. When the fluorescent molecules 14 are excited by the randomly polarized excitation light 230, the fluorescent molecules 14 emit randomly polarized fluorescence 234.

An analyzer 236 transmits components of the randomly polarized fluorescence 234 emitted by the fluorescent molecules that vibrate in a specific direction, and cut off components that vibrate in other directions. In other words, the only light that vibrates in the specific direction passes through the analyzer 236. The component of the fluorescence 234 that vibrates in the specific direction capable of passing through the analyzer 236 is a component which is excited by a component of the excitation light 230 which is linearly polarized in the vibration direction 232a. The vibration direction 232a is the direction of the transition moments (the longitudinal direction of the fluorescent molecule 14 denoted by the oval) of the fluorescent molecules 14 which have been oriented by the laser beam 134, and is perpendicular to the direction of the transition moments of the fluorescent molecules 14 which have been oriented by the laser beam 136. Accordingly, the vibration direction of the fluorescence 234 that passes through the analyzer 236 is substantially only the vibration direction 232a in Figures 17A and 17B. Thereby, only the component of the fluorescence 234 emitted by the fluorescent molecules 14 which is excited by the component of the excitation light 230 which is linearly polarized in the vibration direction 232a reaches a photodiode 238. The component of the excitation light 230 which is linearly polarized in the vibration direction 232a contributes to the emission of the component that vibrates in the vibration direction 232a included in the fluorescence 234. That is, the excitation efficiency of the fluorescent molecules 14 with respect to the component of the excitation light 230 which is linearly polarized in the vibration direction 232a appears as the intensity of fluorescence detected by the photodiode 238. By adopting this configuration, the same measurements as those performed by the first embodiment can be performed with respect to light that vibrates in a specific direction, even if randomly polarized light is employed as the excitation light 230. Note that the component of the fluorescence 234 that vibrates in the specific direction transmitted by the analyzer 236 is not limited to the component that vibrates in the direction described here. A component that vibrates in any direction may be transmitted by the analyzer 236 as long as differences occur in the excitation efficiency of the fluorescent molecules 14 accompanying changes in the orientation direction thereof.

In addition, Figure 17A and Figure 17B illustrate examples in which the amplitude of vibration is constant for all vibration directions. However, it is not necessary for the amplitude of vibration to be constant for all vibration directions. The component of the randomly polarized fluorescence 234 which is received by the photodiode 238 is only that which vibrates in the specific direction, and therefore components that vibrate in other directions are cut off.

As illustrated in Figure 17A, when the orientation control signal is 0V, the direction of the transition moment of the fluorescent molecule 14 which is oriented by the laser beam 136 is perpendicular to the vibration direction 232a of the component which is transmitted through the analyzer 236. In this case, the excitation efficiency of the fluorescent molecule 14 with respect to the component of the excitation light 230 that vibrates in the vibration direction 232a is minimal. Accordingly, the intensity of the component of the fluorescence 234 emitted by the fluorescent molecule 14 that passes through the analyzer 236 and reaches the photodiode 238 in this case is also minimal.

In contrast, as illustrated in Figure 17B, when the orientation control signal is 5V, the direction of the transition moment of the fluorescent molecule 14 which is oriented by the laser beam 134 is parallel to the vibration direction 232a of the component which is transmitted through the analyzer 236. In this case, the excitation efficiency of the fluorescent molecule 14 with respect to the component of the excitation light 230 that vibrates in the vibration direction 232a is maximal. Accordingly, the intensity of the component of the fluorescence 234 emitted by the fluorescent molecule 14 that passes through the analyzer 236 and reaches the photodiode 238 in this case is also maximal.

In the case that such a configuration is adopted as well, the orientation directions of the fluorescent molecules 14 will change when the orientation control signal is switched from 0V to 5V, and the directions of the transition moments of the fluorescent molecules and the vibration direction of light which is transmitted through the analyzer 236 gradually become parallel. Accompanying this gradual approach to becoming parallel, the excitation efficiency of the fluorescent molecules 14 with respect to the component of the excitation light 230 that vibrates in the direction which is capable of being transmitted through the analyzer 236 increases. The increase in excitation efficiency results in an increase of the intensity of the component of the fluorescence 234 emitted by the fluorescent molecules 14 that vibrates in the direction which is capable of being transmitted through the analyzer 236. That is, the intensity of fluorescence detected by the photodiode 238 gradually increases in the same manner as in the first embodiment. In this case, the amounts of time required for orientation of the free molecules and the binding molecules to be completed differ, and therefore the timings at which the intensity of fluorescence received by the photodiode 238 increasing and becoming saturated will differ. For this reason, a graph that represents the output of the photodiode 238 over time when the orientation control signal is switched from 0V to 5V will have the same shape as the graph of Figure 11, even in the case that the configuration described above is adopted. That is, in this case as well, the concentration of the detection target substance can be measured by performing the same calculations as those performed in the first embodiment with respect to the graph representing the output of the photodiode 238.

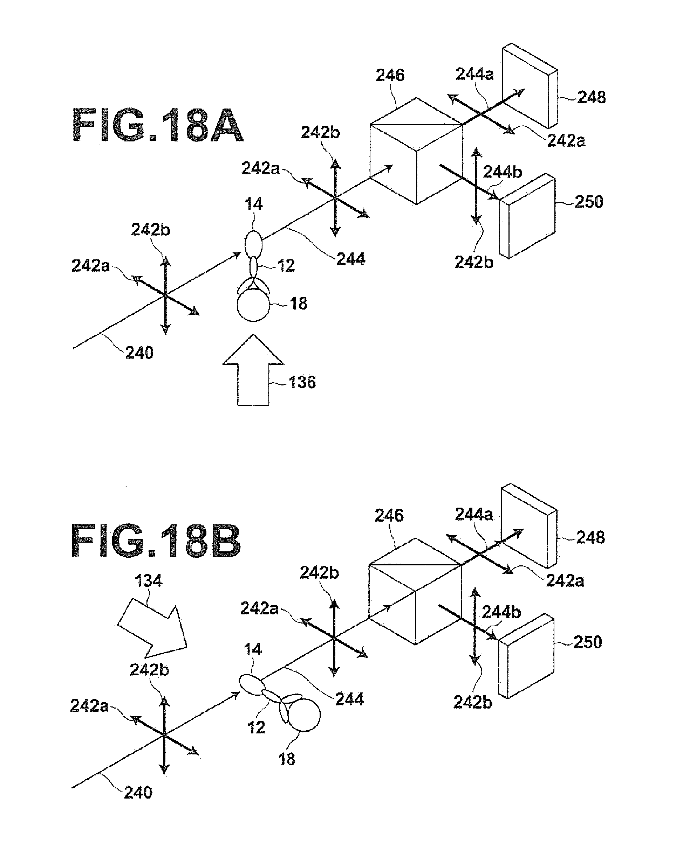

As a further alternative, excitation light 240 that consists of two components which are linearly polarized in two directions perpendicular to each other may be employed, as illustrated in the conceptual diagrams of Figure 18A and Figure 18B. The excitation light 240 only has two components, which are linearly polarized in vibration directions 242a and 242b within a plane perpendicular to the direction in which the excitation light 240 propagates. That is, the vibration direction 242a and the vibration direction 242b are perpendicular to each other. The fluorescent molecules 14 which are excited by the excitation light 240 emit fluorescence 244 having components that vibrate in the same vibration directions as those of the excitation light 240. That is, fluorescence 244 has two components which are linearly polarized in the vibration directions 242a and 242b.

Figure 18A is a conceptual diagram that illustrates a case in which an orientation control signal is 0V. When the orientation control signal is 0V, the laser beam 136 is emitted. The fluorescent molecules 14 which are irradiated by the laser beam 136 become oriented such that the transition moments thereof are the same as the vibration direction 242b. That is, the transition moments of the fluorescent molecules and the vibration direction 242b of one of the components of the excitation light 240 are parallel when the orientation control signal is 0V.

A polarizing beam splitter 246 transmits a linearly polarized component 244a of the fluorescence 244 that vibrates in the vibration direction 242a, and reflects a linearly polarized component 244b of the fluorescence 244 that vibrates in the vibration direction 242b. The linearly polarized component 244a that passes through the polarizing beam splitter 246 reaches a photodiode 248. The linearly polarized component 244b which is reflected by the polarizing beam splitter 246 reaches a photodiode 250.

Figure 18B is a conceptual diagram that illustrates a case in which the orientation control signal is 5V. When the orientation control signal is 5V, the laser beam 134 is emitted. The fluorescent molecules 14 which are irradiated by the laser beam 134 become oriented such that the transition moments thereof are the same as the vibration direction 242a. That is, the transition moments of the fluorescent molecules and the vibration direction 242a of the other one of the components of the excitation light 240 are parallel when the orientation control signal is 5V.

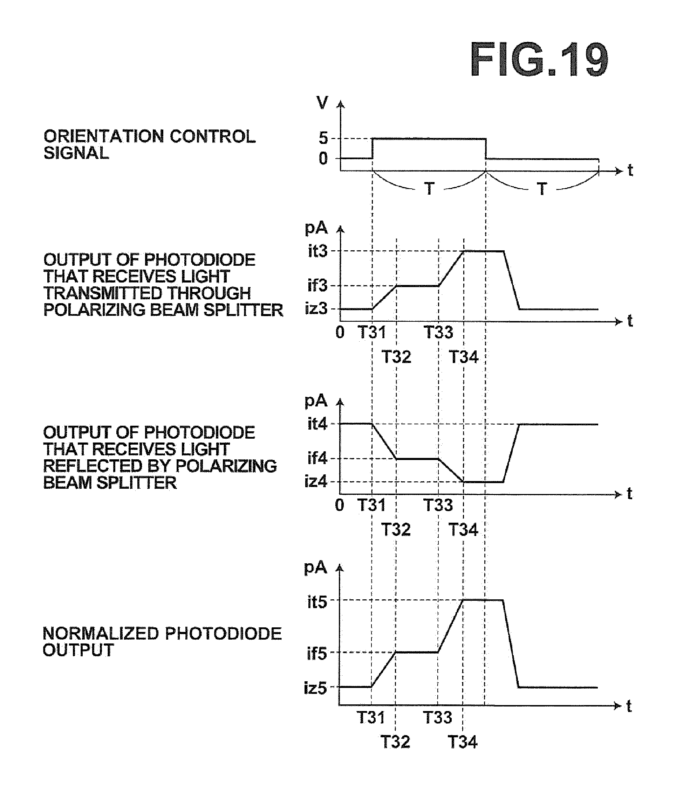

Figure 19 is a collection of graphs having the voltage of an orientation control signal, the output of the photodiode 248, the output of the photodiode 250 and normalized output of the photodiodes as vertical axes, respectively, and time t as the horizontal axes. Note that here, the outputs of the photodiodes are illustrated schematically in the graphs.

In Figure 19, the orientation control signal is set to 0V prior to measurement in the same manner as in the first embodiment. Prior to measurement, the laser beam 136 is irradiated within the solution in the reagent cup, to orient the free molecules and the binding molecules in a single direction. The laser beam 136 is switched to the laser beam 134 accompanying initiation of measurement.

The linearly polarized component 244a of the fluorescence 244 that passes through the polarizing beam splitter 246 will be focused on. In this case, the relationship between the vibrating direction 242a of one of the components of the excitation light 240 and the direction of the transition moments of the fluorescent molecules 14 is the same as in the case of the first embodiment. Temporal changes in the output of the photodiode 248 are similar to those indicated in the graph of Figure 11, which was described with respect to the First embodiment. That is, the orientation directions of the free molecules begin to change accompanying the switch in the emission direction of the laser beam at time T31, and the output of the photodiode 248 increases from an initial value iz3. The output of the photodiode 248 becomes a value if3 at time T32 after reorientation of the free molecules is complete, and remains constant for a period of time thereafter. Then, the output of the photodiode 248 increases again at time T33, when reorientation of the binding molecules begins. Thereafter, the output of the photodiode 248 becomes a maximal value it3 at time T34, when the reorientation of the binding molecules is complete. The orientation control signal is reset to 0V after maintaining a voltage of 5V for T seconds. When the orientation control signal is switched from 5V to 0V, the output of the photodiode 248 remains at the value it3 for a period of time, and then decreases to the value iz3.

The linearly polarized component 244b of the fluorescence 244 which is reflected by the polarizing beam splitter 246 will be focused on. In this case, the vibrating direction 242b of the other one of the components of the excitation light 240 and the direction of the transition moments of the fluorescent molecules 14 is parallel until time T31. Therefore, the excitation efficiency of the fluorescent molecules 14 with respect to the other component of the excitation light 240 is maximal until time T31. That is, the intensity of the linearly polarized component 244b of the fluorescence 244 is maximal until time T31, and therefore the output of the photodiode 250 that receive the linearly polarized component 244b of the fluorescence 244 reflected by the polarizing beam splitter 246 is also maximal until time T31. The orientation directions of the free molecules begin to change accompanying the switch in the emission direction of the laser beam at a time T31, and the output of the photodiode 250 decreases from an initial value it4. The output of the photodiode 250 becomes a value if4 at time T32 after reorientation of the free molecules is complete, and remains constant for a period of time thereafter. Then, the output of the photodiode 250 decreases again at time T33, when reorientation of the binding molecules begins. Thereafter, the output of the photodiode 250 becomes a minimal value iz4 at time T34, when the reorientation of the binding molecules is complete. The orientation control signal is reset to 0V after maintaining a voltage of 5V for T seconds. When the orientation control signal is switched from 5V to 0V, the output of the photodiode 250 remains at the value iz4 for a period of time, and then increases to the value it4. This is because the vibration direction 242b of the other component of the excitation light 240 and the directions of the transition moments of the fluorescent molecules 14 return to being in a parallel state.

The CPU designates the output of the photodiode 248 as Pp and the output of the photodiode 250 to Pv, and normalizes these values according to Formula (3) below.

$$K=(Pp-Pv)/(Pp+Pv) \qquad (3)$$

The influence of fluctuations in the concentrations of the free molecules and the binding molecules and fluctuations in the excitation power of the optical systems can be reduced, by normalizing the outputs of the two photodiodes in this manner.

Then, the concentration of the binding molecules is calculated from the graph of the normalized output of the photodiodes. Specifically, a measurement value S3 is obtained by Formula (4) below.

$$S3=(it5-if5)/(it5-iz5) \qquad (4)$$

A calibration curve function is employed in the same manner as in the first embodiment, to obtain a diagnosis value C3 (the concentration of the detection target substance) from the obtained measurement value S3.

The embodiments of the present invention were described as cases in which the directions of the transition moments of the fluorescent molecules are controlled by switching the direction in which the laser beam is emitted. However, the method for controlling the directions of the transition moments of the fluorescent molecules is not limited to this configuration. For example, the directions of the transition moments of the fluorescent molecules may be controlled by controlling the vibration direction of a linearly polarized laser beam, utilizing the phenomenon that the transition moments of fluorescent molecules track the vibration direction of linearly polarized light.

An example of a method for controlling the directions of the transition moments of the fluorescent molecules by controlling the vibration direction of a linearly polarized laser beam will be described. A laser beam which is linearly polarized in a single direction is employed, and the polarization axis of the laser beam is rotated to control the orientations of the free molecules and the binding molecules, thereby controlling the directions of the transition moments of the fluorescent molecules. The free molecules or the binding molecules on which the linearly polarized laser beam is emitted will be oriented in a specific direction which is determined by the polarization axis of the laser beam. The polarization axis of the linearly polarized laser beam may be controlled by employing a $\lambda/2$ wavelength plate. $\lambda/2$ wavelength plates are phase plates that function to cause optical path differences between two perpendicular components of light to half the wavelength thereof, and are employed to rotate the polarization axes of light. Light which is linearly polarized in a direction parallel to the direction of the optical axis of a $\lambda/2$ wavelength plate passes therethrough as is, whereas light which is linearly polarized in a direction that forms a 45 degree angle with the direction of the optical axis of a $\lambda/2$ wavelength plate is transmitted in a state in which the polarization axis thereof is rotated 90 degrees. That is, switching between a case in which a laser beam passes through the $\lambda/2$ wavelength plate as is and a case in which a laser beam is transmitted in a state in which the polarization axis thereof is rotated 90 degrees is enabled, by switching the angle of the $\lambda/2$ wavelength plate with respect to the linearly polarized laser beam. That is, the free molecules and the binding molecules can be oriented in two directions by rotating the polarization axis of the linearly polarized laser beam employing the $\lambda/2$ wavelength plate.

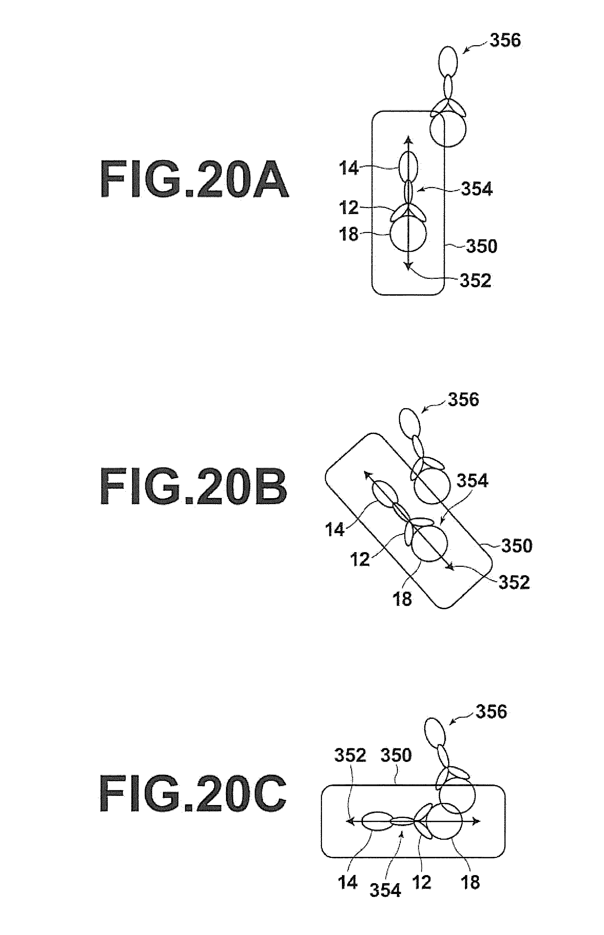

In the case that the directions of the transition moments of the fluorescent molecules are controlled by controlling the vibration direction of the linearly polarized laser beam, the laser beam may be of any cross sectional shape within a plane perpendicular to the propagation direction thereof. For example, a case will be considered in which a linearly polarized laser beam 350 having a polarization axis 352 is emitted, as illustrated in Figure 20A. In this case, the laser beam 250 has a substantially rectangular cross sectional shape in a direction perpendicular to the propagation direction thereof. The motions of a binding molecule 354 positioned at the center of the laser beam 350 and a binding molecule 356 positioned at the peripheral portion of the laser beam 350 will be considered.

As illustrated in Figure 20B, the polarization axis 352 rotates when the laser beam 350 is rotated. The binding molecule 354 which is positioned on the rotational axis (the center of rotation of the polarization axis 352) immediately tracks the rotation of the polarization axis 352, and rotates. Meanwhile, the binding molecule 356 positioned at the peripheral portion of the laser beam 350 cannot track the rotation of the polarization axis 352 immediately, and becomes separated therefrom. After a period of time, the binding molecule 356 is also drawn into the laser beam 350, and initiates rotation that tracks the rotation of the polarization axis 352. In the case that the polarization axis 352 of the laser beam 350 is rotated 90 degrees from the polarization axis 352 of Figure 20A as illustrated in Figure 20C, the reorientation of the binding molecule 354 is completed simultaneously with the completion of rotation of the polarization axis 352. Meanwhile, because the binding molecule 356 cannot track the rotation of the polarization axis 352 immediately, reorientation of the binding molecule 356 is completed after a period of time following completion of rotation of the polarization axis 352. That is, the movement of the binding molecule 354 positioned on the rotational axis is rotation which is synchronized with the rotation of the polarization axis 352 of the laser beam 350. However, the movement of the binding molecule 356 positioned at the peripheral portion of the laser beam 350 is revolution about the rotational axis which is not synchronized with the rotation of the polarization axis 352 of the laser beam 350.

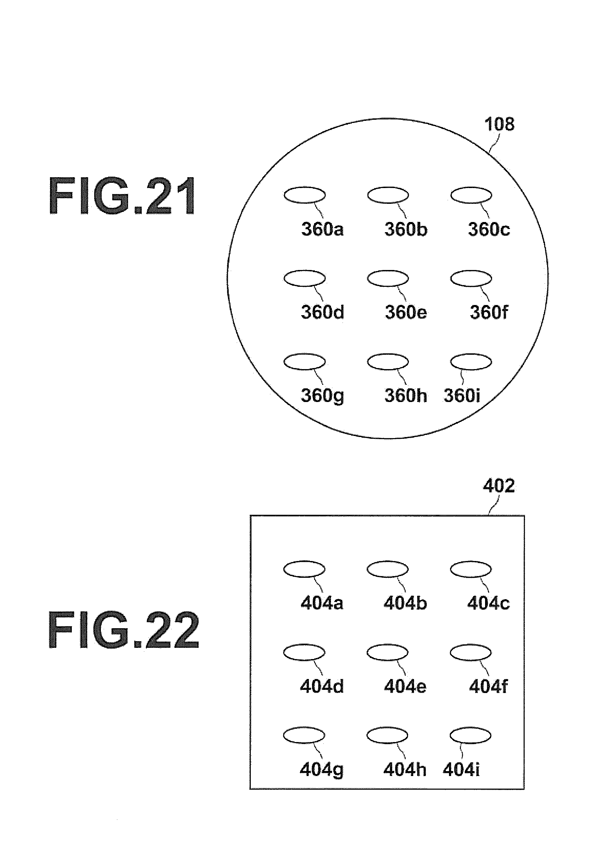

There are cases in which the presence of binding molecules that cannot track the rotation of the polarization axis 352 of the laser beam 350 will influence measurements. In order to reduce such influence, it is preferable for the laser beam to simultaneously enter a plurality of points from a predetermined direction. For example, as illustrated in Figure 21 (a plan view of the reagent cup 108) a configuration may be adopted, in which nine laser beams corresponding to nine points 360a through 360i enter the reagent cup 108. By adopting such a configuration, the number of binding molecules which are positioned at the center of the polarization axes of the laser beams will increase, thereby reducing the aforementioned influence on measurements. Note that although an example in which laser beams enter nine points is described here, the number of points that the laser beams enter is not limited to nine, and may be greater or less than nine. It is desirable for laser beams to enter a greater number of points the narrower that they are focused. Thereby, the binding molecules can be caused to rotate in synchrony with the rotation of the laser beams. As a result, sudden variations in fluorescent intensity can be reduced, and the coefficient of variation, which is an index that represents relative spreading, can be improved.

The structure of an orientation controlling light source 402 that causes laser beams to simultaneous enter a plurality of points from a predetermined direction is illustrated in Figure 22. The orientation controlling light source 402 is a 3·3 two dimensional laser array. Nine light emitting points 404a through 404i of the orientation controlling light source 402 emit light. The light emitting points have heights of $1\mu m$ and widths of $100\mu m$. The distances among the light emitting points are approximately $100\mu m$.

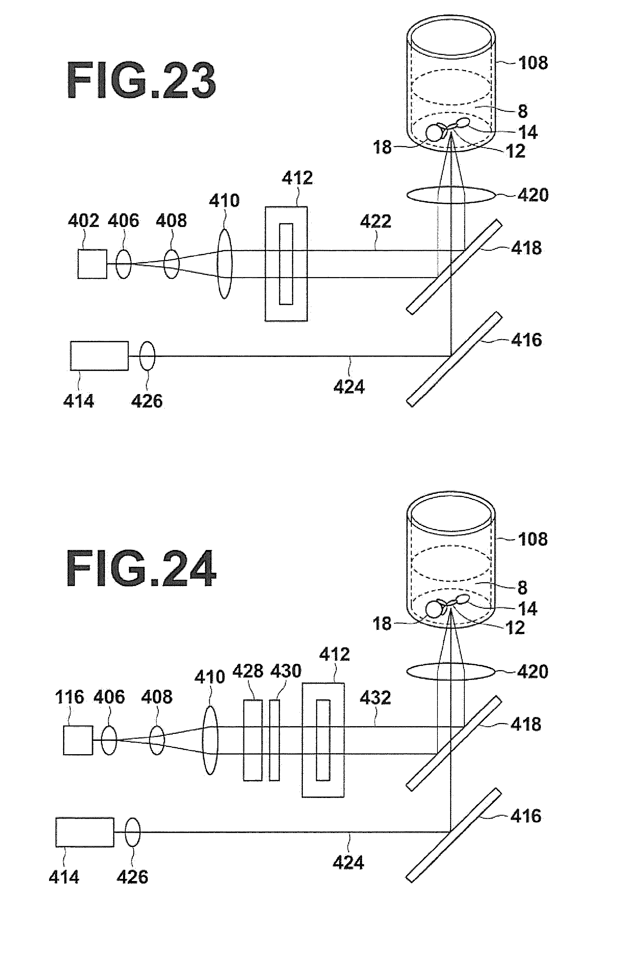

An example of an optical system that employs the orientation controlling light source 402 of Figure 22 is illustrated in Figure 23. Note that structural elements other than the optical systems for laser beams and excitation light are omitted in Figure 23.

Linearly polarized laser beams 422 output from the orientation controlling light source 402 pass through a collimating lens 406 and become collimated light beams at a focal point. The laser beams 422 which have passed through the collimating lens 406 pass through beam expanders 408 and 410, then enter a λ/2 wavelength plate 412. The laser beams 422 which have passed through the beam expanders 408 and 410 are spread to become a collimated light beam having a specific magnification ratio. The λ/2 wavelength plate is on a rotatable stage, and is configured to be rotatable. This configuration enables the vibration direction of the laser beams 422 to be rotated. The laser beams 422 which have passed through the λ/2 wavelength plate are reflected by a dichroic mirror 418, focused by a lens 420, enter the reagent cup 108 through the bottom surface thereof, and propagate upward.

Excitation light 424 output from a light source 414 passes through a lens 426 and is reflected by a dichroic mirror 416. The excitation light 424 which has been reflected by the dichroic mirror 416 passes through a dichroic mirror 418, is focused by a lens 420, enters the reagent cup 108 through the bottom surface thereof, and propagates upward.

If the focal distance of the collimating lens 406 is set to be 3.1mm, and the focal distance of the lens 420 is set to be 4mm in the optical system illustrated in Figure 23, the magnification ratio will be 1.29x. Therefore, the sizes of the laser beams 422 are approximately $1.3\mu m \cdot 130\mu m$ with pitches of approximately $129\mu m$ at the bottom surface of the reagent cup 108.

Another example of an optical system that causes laser beams to simultaneously enter a plurality of points from a predetermined direction will be described with reference to Figure 24. Note that structural elements other than the optical systems for laser beams and excitation light are omitted in Figure 24. In addition, structural elements which are the same as those illustrated in Figure 23 are denoted by the same reference numerals, and detailed descriptions thereof will be omitted.

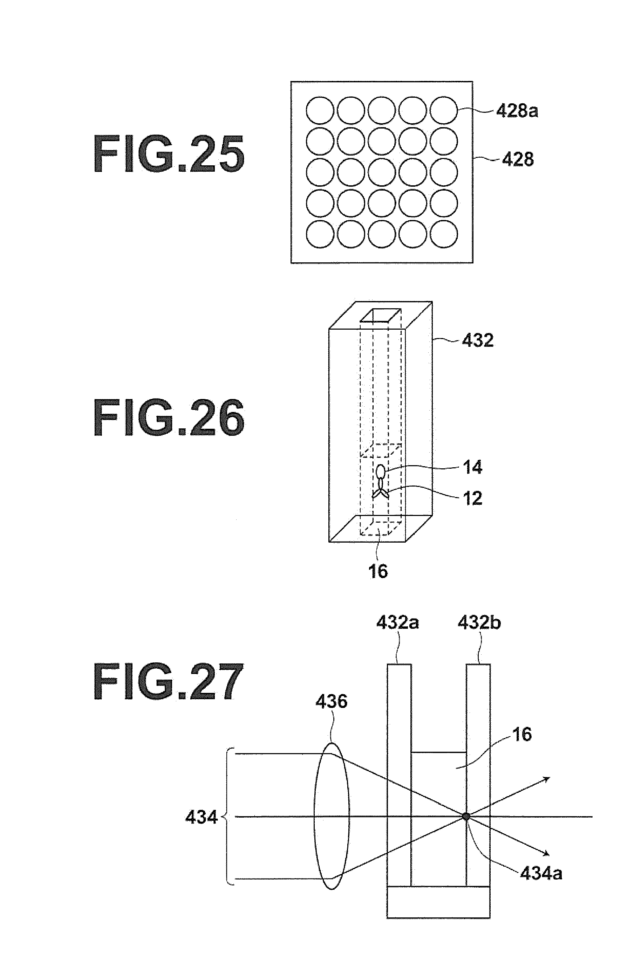

In the optical system illustrated in Figure 24, the orientation controlling light source 116 is the same as that of the first embodiment. A laser beam 432 passes through the collimating lens 406, the beam expanders 408 and 410, and enters a microlens array 428. As illustrated in Figure 25, the microlens array 428 has a plurality of microlenses 428a arrayed in a lattice shape. The laser beam 432 which passes through the microlens array 428 becomes a plurality of light beams which have different focal points, as light emitted by a plurality of light sources. The laser beam 432 is focused by a pinhole array 430, reflected by the dichroic mirror 418, focused by the lens 420, enters the reagent cup 108 through the bottom surface thereof, and propagates upward. Laser beams can be caused to simultaneously enter a plurality of points from a predetermined direction by employing a microlens array in this manner as well.

Examples in which the λ/2 wavelength plate 412 is employed to change the vibration direction were described. Alternatively, a liquid crystal phase modulating device which is controlled by electrical signals maybe employed to change the vibration direction.

In addition, the reagent cup was of a cylindrical shape in the embodiments described above. However, it is not necessary for the shape of the reagent cup to be cylindrical. For example, a reagent cup 432 shaped as a rectangular column and having a rectangular columnar solution portion therein may be employed, as illustrated in Figure 26. The reagent cup 432 having the rectangular columnar solution portion is particularly suited in cases that pressure applied by the laser beam in the propagation direction thereof is utilized to press the free molecules and the binding molecules against the surface of an inner wall of the reagent cup 432. This is a phenomenon that occurs in cases that the masses of the free molecules and the binding molecules are light, caused by the free molecules and the binding molecules moving through the solution under the pressure applied by the laser beam. In this case, if the solution holding portion is a rectangular column, the free molecules and the binding molecules are oriented while being pressed against the interface between the solution and the reagent cup 432. In the case that the interface is a flat surface and the pressure applied by the laser beam operates in a direction perpendicular to the interface, the free molecules and the binding molecules will not move outside the irradiation range of the laser beam by moving in directions parallel to the interface.

In addition, in the case that the free molecules and the binding molecules are pressed against the surface of the inner wall of the reagent cup 432, the molecules can be more easily oriented by setting the position of the focal point of the laser beam. Figure 27 is a diagram that illustrates the positional relationship between the focal point of a focused laser beam and a reagent cup. A laser beam 434 enters a lens 436 and is focused at a focal point 434a at the interface between the plasma 16 and a side wall 432b (the inner surface of the side wall 432b). The intensity of the laser 434 is greatest at the position of the focal point 434a, and therefore the free molecules and the binding molecules can be pressed with a great amount of pressure. Accordingly, if the laser 434 is emitted in the manner illustrated in Figure 27, the free molecules and the binding molecules can be more efficiently oriented while pressing the free molecules and the binding molecules against the inner surface of the side wall 432b. In this case as well, the orientation directions of the free molecules and the binding molecules can be changed at the position of the focal point 434a by rotating the vibration direction of the linearly polarized laser beam 434.

Note that it is not necessary for the solution holding portion to be shaped as a rectangular column, and the solution holding portion needs only to have at least one flat surface. If a laser beam is emitted such that it is focused at a focal point on the flat surface, the free molecules and the binding molecules will not move outside the irradiation range of the laser beam by moving in directions parallel to the flat surface, and will be oriented while being pressed against the flat surface.

[Field of Industrial Applicability]

The biological molecule detecting apparatus and the biological molecule detecting method of the present invention may be utilized in apparatuses that detect or quantify detection target substances by utilizing interactions between the detection target substances and substances that specifically bind to the detection target substances.

**Claims**

1. A biological molecule detecting apparatus that causes a detection target substance, a specific binding substance that specifically binds to the detection target substance, and fluorescent molecules to bind to each other within a solution, and detects fluorescence emitted by the fluorescent molecules to detect or quantify the detection target substance, **characterized by** comprising:

   a light source that emits excitation light having a light component which is linearly polarized in a specific direction that excites the fluorescent molecules;
   orientation control means for switching the orientation of the fluorescent molecules within the solution to orientations in at least two directions;
   a light receiving section that detects the fluorescence emitted by the fluorescent molecules; and
   a calculating section that detects or quantifies the detection target substance based on the fluorescence detected by the light receiving section.

2. A biological molecule detecting apparatus as defined in Claim 1, **characterized by**:

   the orientation control means switching the orientation of the fluorescent molecules between an orientation in a first direction in which the direction of the transition moments of the fluorescent molecules and the vibration direction of the excitation light are parallel, and an orientation in a second direction in which the direction of the transition moments of the fluorescent molecules and the vibration direction of the excitation light are perpendicular.

3. A biological molecule detecting apparatus as defined in either one of Claim 1 and Claim 2, **characterized by**:

   the orientation control means switching the orientation of the fluorescent molecules at predetermined temporal intervals;
   the light receiving section detecting fluorescence a plurality of times; and
   the calculating section calculating an arithmetic mean of a plurality of detected fluorescent intensities, and detects or quantifies the detection target substance based on the arithmetic mean of the fluorescent intensities.

4. A biological molecule detecting apparatus as defined in Claim 3, **characterized by**:

   the predetermined temporal intervals being determined based on the mass or the volume of the detection target substance, the specific binding substance, and the fluorescent molecules, and the degree of orientation control exerted by the orientation control means.

5. A biological molecule detecting apparatus as defined in any one of Claims 1 through 4, **characterized by**:

   the orientation control means controlling the orientation of the fluorescent molecules by emitting light having a wavelength different from that of the excitation light.

6. A biological molecule detecting apparatus as defined in Claim 5, **characterized by**:

   the orientation control means being that which emits light having a wavelength different from that of the excitation light onto the solution from a plurality of positions.

7. A biological molecule detecting apparatus as defined in either one of Claim 5 and Claim 6, **characterized by**:

the solution being held in a solution holding portion having a flat surface at least at a portion thereof.

8. A biological molecule detecting apparatus as defined in Claim 7, **characterized by**:

the orientation means being that which emits light having a wavelength different from that of the excitation light in a direction that passes through the solution and exits the flat surface of the solution holding portion such that the light is conjugately focused with the excitation light emitted by the light source at an interface between the solution and the flat surface.

9. A biological molecule detecting apparatus as defined in any one of Claims 1 through 8, **characterized by**:

the light receiving section being equipped with spectral means for spectrally separating light.

10. A biological molecule detecting apparatus as defined in Claim 9, **characterized by**:

the spectral means being a plurality of filters having different properties; and
the light receiving section switching a filter to be employed from among the plurality of filters according to the light emitting wavelength of the fluorescent molecules.

11. A biological molecule detecting apparatus as defined in any one of Claims 1 through 10, **characterized by**:

the calculating section detecting or quantifying the detection target substance by utilizing the fact that there are differences in the temporal changes in the intensity of fluorescence emitted by the fluorescent molecules which are bound to the detection target substance via the specific binding substance and the temporal changes in the intensity of fluorescence emitted by the fluorescent molecules which are not bound to the detection target substance during the switch of the orientation by the orientation control means from a first direction to a second direction.

12. A biological molecule detecting method for causing a detection target substance, a specific binding substance that specifically binds to the detection target substance, and fluorescent molecules to bind to each other within a solution, and detects fluorescence emitted by the fluorescent molecules to detect or quantify the detection target substance, **characterized by** comprising the steps of:

emitting excitation light having a light component which is linearly polarized in a specific direction that excites the fluorescent molecules;
switching the orientation of the fluorescent molecules within the solution to orientations in at least two directions;
detecting the fluorescence emitted by the fluorescent molecules; and
detecting or quantifying the detection target substance based on the detected fluorescence.

FIG.1A

FIG.1B

# FIG.2A

19

14

12

PROPAGATING DIRECTION
OF EXCITATION LIGHT

TRANSITION MOMENT

VIBRATING DIRECTION
OF EXCITATION LIGHT

# FIG.2B

19

12    14

PROPAGATING DIRECTION
OF EXCITATION LIGHT

TRANSITION
MOMENT

VIBRATING DIRECTION
OF EXCITATION LIGHT

# FIG.3A

12    14

# FIG.3B

18    12    14

FIG.4A

106

100

102

104

FIG.4B

106

100

108

102

110

104

# FIG.5

EP 2 579 024 A1

16

108

136

138

119

EXCITATION
LIGHT
SOURCE

118

140

LIGHT
SHIELDING
PLATE

134

136

120

AOD

FG

117

122

ORIENTATION
CONTROLLING
LIGHT SOURCE

116

# FIG.6

EP 2 579 024 A1

# FIG.7A

12
14

ORIENTATION DIRECTION
OF MOLECULE

134

# FIG.7B

136

ORIENTATION DIRECTION
OF MOLECULE

14

12

29

**FIG.8A**

108

134

18    12    14

16

**FIG.8B**

108

16

14

12

18

136

FIG.9

108

16

14

18

12

142

124

144

146

148

147

149

PD

150

TO AMPLIFIER 126

FIG.10

EP 2 579 024 A1

# FIG.11

ORIENTATION
CONTROL SIGNAL

SAMPLING CLOCK

PHOTODIODE
OUTPUT

A/D CONVERTING
SECTION OUTPUT

EP 2 579 024 A1

# FIG.12

ORIENTATION
CONTROL
SIGNAL

FIG.13A

FIG.13B

# FIG.14

EP 2 579 024 A1

FIG.15

# FIG.16A

# FIG.16B

# FIG.17A

# FIG.17B

FIG.18A

FIG.18B

# FIG.19

ORIENTATION CONTROL SIGNAL

OUTPUT OF PHOTODIODE THAT RECEIVES LIGHT TRANSMITTED THROUGH POLARIZING BEAM SPLITTER

OUTPUT OF PHOTODIODE THAT RECEIVES LIGHT REFLECTED BY POLARIZING BEAM SPLITTER

NORMALIZED PHOTODIODE OUTPUT

**FIG.20A**

356
14
354
12
18
350
352

**FIG.20B**

356
354
14
12
350
18
352

**FIG.20C**

356
352  350
14  354  12  18

**FIG.21**

108

360a  360b  360c

360d  360e  360f

360g  360h  360i

402

**FIG.22**

404a  404b  404c

404d  404e  404f

404g  404h  404i

# FIG.23

108

8

14

18

12

412

410

402 406 408

422

420

418

414 426

416

424

# FIG.24

108

8

14

18

12

412

410 428 430

432

116 406 408

420

418

414 426

416

424

# FIG.25

428a

428

# FIG.26

432

14

12

16

# FIG.27

432a    432b

436

16

434

434a

## INTERNATIONAL SEARCH REPORT

| | International application No. |
|---|---|
| | PCT/JP2011/003140 |

| | |
|---|---|
| **A. CLASSIFICATION OF SUBJECT MATTER** | |
| *G01N21/64*(2006.01)i, *G01N33/53*(2006.01)i, *G01N33/536*(2006.01)i | |

According to International Patent Classification (IPC) or to both national classification and IPC

| | |
|---|---|
| **B. FIELDS SEARCHED** | |
| Minimum documentation searched (classification system followed by classification symbols) | |
| G01N21/00-21/74 | |

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2011 |
| Kokai Jitsuyo Shinan Koho | 1971-2011 | Toroku Jitsuyo Shinan Koho | 1994-2011 |

Electronic data base consulted during the international search (name of data base and, where practicable, search terms used)
JSTPlus/JMEDPlus/JST7580(JDreamII)

**C. DOCUMENTS CONSIDERED TO BE RELEVANT**

| Category* | Citation of document, with indication, where appropriate, of the relevant passages | Relevant to claim No. |
|---|---|---|
| Y | JP 61-272637 A (Hitachi, Ltd.), 02 December 1986 (02.12.1986), entire text; all drawings (Family: none) | 1,9-10,12 |
| Y | JP 2003-315258 A (Aisin Seiki Co., Ltd.), 06 November 2003 (06.11.2003), paragraphs [0045], [0062] (Family: none) | 1,9-10,12 |
| Y | JP 2000-19172 A (Matsushita Electric Industrial Co., Ltd.), 21 January 2000 (21.01.2000), entire text; all drawings & US 6448018 B1 & EP 969279 A2 & DE 69912361 D | 9-10 |

| | | | |
|---|---|---|---|
| ☒ Further documents are listed in the continuation of Box C. | | ☐ See patent family annex. | |

| | | |
|---|---|---|
| * Special categories of cited documents: | "T" | later document published after the international filing date or priority date and not in conflict with the application but cited to understand the principle or theory underlying the invention |
| "A" document defining the general state of the art which is not considered to be of particular relevance | | |
| "E" earlier application or patent but published on or after the international filing date | "X" | document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" document referring to an oral disclosure, use, exhibition or other means | | |
| "P" document published prior to the international filing date but later than the priority date claimed | "&" | document member of the same patent family |

| | |
|---|---|
| Date of the actual completion of the international search | Date of mailing of the international search report |
| 16 August, 2011 (16.08.11) | 30 August, 2011 (30.08.11) |
| Name and mailing address of the ISA/ | Authorized officer |
| Japanese Patent Office | |
| Facsimile No. | Telephone No. |

Form PCT/ISA/210 (second sheet) (July 2009)

| INTERNATIONAL SEARCH REPORT | International application No. |
|---|---|
| | PCT/JP2011/003140 |

C (Continuation).   DOCUMENTS CONSIDERED TO BE RELEVANT

| Category* | Citation of document, with indication, where appropriate, of the relevant passages | Relevant to claim No. |
|---|---|---|
| Y | JP 62-38346 A  (Hitachi, Ltd.), 19 February 1987 (19.02.1987), entire text; all drawings (Family: none) | 9-10 |
| A | JP 10-104079 A  (Laboratory of Molecular Biophotonics), 24 April 1998 (24.04.1998), entire text; all drawings (Family: none) | 1-12 |
| A | WO 2003/024586 A1  (National Institute of Advanced Industrial Science and Technology), 27 March 2003 (27.03.2003), entire text; all drawings & US 2004/0239854 A1    & EP 1426103 A2 | 5-8 |

Form PCT/ISA/210 (continuation of second sheet) (July 2009)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 7120397 A

- JP 2008298743 A